(19) Europäisches Patentamt
European Patent Office
Office européen des brevets

(11) Publication number: **0 459 471 A1**

(12) # EUROPEAN PATENT APPLICATION

(21) Application number: **91108837.5**

(22) Date of filing: **29.05.91**

(51) Int. Cl.5: **C07C 29/15**, C07C 31/04, C07C 27/00, B01J 19/08

(30) Priority: **01.06.90 JP 144227/90**

(43) Date of publication of application: **04.12.91 Bulletin 91/49**

(84) Designated Contracting States: **DE FR GB NL**

(71) Applicant: **ADVANTEST CORPORATION**
**32-1, Asahi-cho 1-chome**
**Nerima-Ku Tokyo(JP)**

(72) Inventor: **Watanabe, Masao**
**5-5, Nijo 5-chome, Sumikawa, Minami-ku**
**Sapporo-shi, Hokkaido(JP)**

(74) Representative: **Blumbach Weser Bergen Kramer Zwirner Hoffmann Patentanwälte**
**Radeckestrasse 43**
**W-8000 München 60(DE)**

(54) **Method for photosynthesis of organic substances from carbon dioxide and water.**

(57) Photosynthesis of organic substances from $CO_2$ gas is carried out in such a manner that: ZnO powder (11) is activated in its surface by a heat treatment; said activated ZnO powder is supplied with $CO_2$ gas and $H_2O$ gas and is simultaneously irradiated by visible light to photosynthesize organic substances, of which major product is methanol. The present inventive method is free from hydrogen gas as well as UV rays so that it is quite economical and is practically applicable to industrial purposes.

FIG. 1

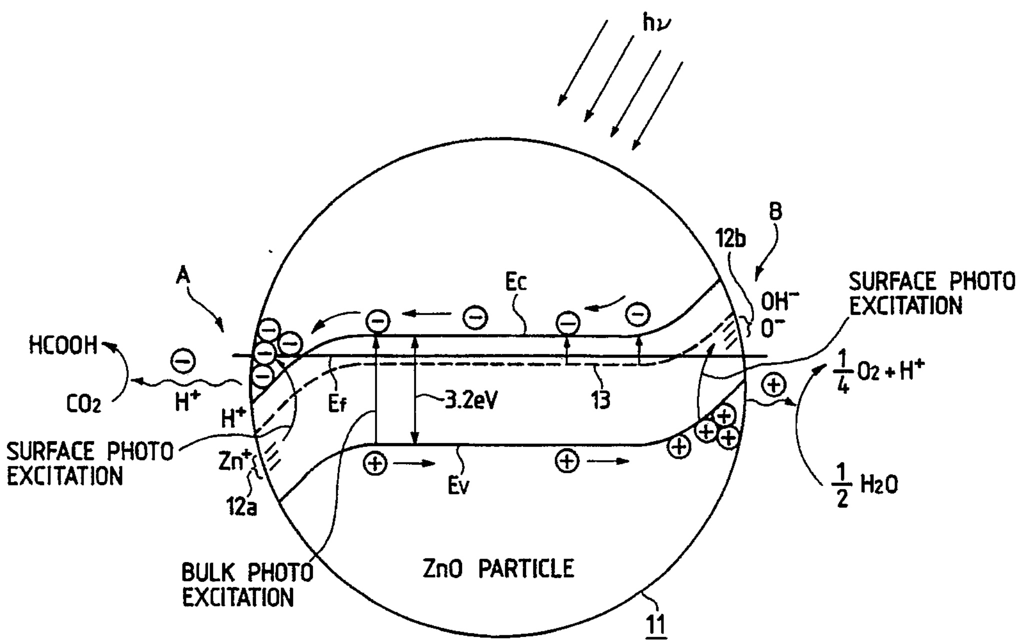


EP 0 459 471 A1

## BACKGROUND OF THE INVENTION

The present invention relates to a synthesis of primitive organic substances such as methanol through photosynthetic reaction of carbon dioxide with water.

The restriction of carbon dioxide amount exhausted in air has been proposed for purposes of terrestrial environment preservation and therewith studies on reduction and conversion of $CO_2$ molecules have been progressing increasingly. The trend of such studies at present is on reactions of decomposing $CO_2$ molecules or of decomposing and simultaneously compounding into organic molecules, and there are, for example, catalytic reactions, organic synthesis, electrochemical reactions, and photoelectric chemical reactions, etc. One of the above reactions has been known as a Fischer-Tropsch reaction, in which carbon dioxide gas is decomposed into carbon monoxide and oxygen and then the former is reacted with hydrogen through a catalytic reaction to give hydrocarbons having large molecular weights. This method, however, is industrially disadvantageous for practical use because it requires a large quantity of expensive hydrogen gas and hydrocarbons yielded are rather expensive and furthermore high-safety equipment is needed because of a danger of explosive hydrogen gas, which results in high cost. Separately, catalytic reactions of synthesizing methanol or methane from $CO_2$ and $H_2$ molecules have been studied but they seem to be disadvantageous because of their expensive raw material, $H_2$.

Studies on a photosynthesis which is considered to be the simplest, are expressed as the following formula but they are in an initial stage yet.

$$CO_2 + 2H_2O \xrightarrow[\text{cat.}]{h\nu} CH_3OH + \frac{3}{2} O_2 - 63 \text{ kcal/mol} \qquad (1)$$

Fujishima et al. had reported on this photosynthesis using various semiconductors as a catalyst. (Nature Vol. 277, Feb. 1979, pp 637-638). They selected powders of $TiO_2$, ZnO, CdS, Gap, SiC and $WO_3$, etc., as semiconductor catalysts and conducted experiments, in which each powder was suspended in water respectively and the resultant mixtures were respectively illuminated by a Xe lamp while bubbling $CO_2$ gas therein for 7 hours, for example. Yield of methanol in slight amount was confirmed in every catalyst except for $WO_3$ and particularly SiC was found yielded a larger amount than others. However, Fujishima et al. method has such drawbacks as follows: the amount of methanol yielded is very slight; as a light source required for photosynthesis, ultraviolet rays must be employed, which are little included in the sun light on earth surface, so that the use of UV light source like a Xe lamp becomes essential resulting in impracticability and high cost; and the photosynthesis is carried out in water, which means that apparatuses to separate the products from water are necessary.

## SUMMARY OF THE INVENTION

An object of the invention is to provide a method of photosynthesizing organic substances from $CO_2$ gas, in which $CO_2$ gas is converted safely into primitive organic substances in a relatively cheap, simple way and moreover the productant organic substances can be easily discharged.

In accordance with the present inventive method, metal oxides are heated to be activated in their surfaces and $CO_2$ and $H_2O$ gas are supplied thereto and then by irradiating visible light, organic substances are photosynthesized from said $CO_2$ and $H_2O$ gas.

As is explained above the present invention can photosynthesize organic substances from $CO_2$ and $H_2O$ gas without using hydrogen gas as a raw material so that it is not only safe but also economical. Furthermore in contrast with the gaseous raw material, the product, for example methanol, is yielded as a fluid, which can be easily discharged.

## BRIEF EXPLANATION OF DRAWINGS

Fig. 1 is a schematic drawing of the principle of the present invention showing a band structure of ZnO fine particle.

Fig. 2 is a block diagram of a vacuum•gas-dose system to be used to prosecute the present inventive principle.

Fig. 3 is a graph showing an IR absorption spectrum originating from stretching vibration of $CO_2$

adsorbed to ZnO.

Fig. 4 is a graph showing an IR absorption spectrum originating from stretching vibration of adsorbed $CO_2$ measured through a KBr window.

Fig. 5 is a graph showing changes of IR absorption spectrum originating from stretching vibration of absorbed $CO_2$, caused by a sun light irradiation.

Fig. 6A is a graph showing an influence of a white Xe lamp irradiation upon the stretching vibration mode of the adsorbing $CO_2$.

Fig. 6B is a graph of IR absorption spectrum characteristic showing a formation of organic substances after the light irradiation.

Fig. 7A is a graph of IR absorption spectrum of ZnO powder after a supply of $CO_2$ + $H_2O$, measured through a KBr window prior to the sun light irradiation.

Fig. 7B is a graph of IR absorption spectrum after 2-hour-irradiation of the sun light.

Fig. 8 is a graph showing a change in the surface conductance of the ZnO single crystals caused by the gas supply.

Fig. 9A is a graph showing a surface photoconductance when ZnO is irradiated by the light of $\lambda$ = 397 nm under vacuum.

Fig. 9B is a graph showing a surface photoconductance of ZnO under $CO_2$ gas of 150 Torr.

Fig. 9C is a graph showing a surface photoconductance of ZnO when supplied by $CO_2$ (150 Torr) + $H_2O$ (20 Torr).

Fig. 10A is a graph showing a surface photoconductance of ZnO when irradiated by the light of $\lambda$ = 500 nm under vacuum.

Fig. 10B is a graph showing a surface photoconductance of ZnO under $CO_2$ gas.

Fig. 10C is a graph showing a surface photoconductance of ZnO when supplied by $CO_2$ + $H_2O$.

Fig. 11 is a graph showing the wavelength dependency of the surface photoconductance under the respective conditions.

Fig. 12 is a table of results obtained by analyzing impurities in ZnO surface.

Fig. 13 is a mass spectrometric graph of photoreaction products yielded by the sun light irradiation in December.

Fig. 14 is a mass spectrometric graph of photoreaction products yielded by the sun light irradiation in June.

Fig. 15 is a mass spectrometric graph of photoreaction products yielded by the Xe lamp UV light irradiation.

Fig. 16 is a mass spectrometric graph of products dissociated from ZnO at the time of UV light irradiation.

Fig. 17 is a mass spectrometric graph of products dissociated from ZnO at the time of visible light irradiation.

Fig. 18 is a graph showing a pressure drop originating from photosynthesis by the visible light.

Fig. 19 is an example of equipment to implement the present inventive method.

## DETAILED DESCRIPTION OF THE PREFERRED EMBODIMENTS

[Principle of the Invention]

In the present invention a semiconductor of metal oxide is used as a catalyst in photosynthesis just like in Fujishima et al., but the feature of the present invention is that said metal oxide is heated to be activated to locally generate free electrons and free holes in its surface, and that thus activated metal oxide is supplied with $CO_2$ and $H_2O$ gases and is simultaneously irradiated by the visible light energy to photosynthesize organic substances from the supplied $CO_2$ and $H_2O$ gases.

As a metal oxide ZnO (zinc oxide) is recommended due to its superior catalytic action in methanol synthesis, and particularly powdery ZnO is preferable so as to obtain a wider catalytic surface. Heating of ZnO fine particles is preferably done under vacuum. When ZnO fine particles are heated under vacuum relatively a great number of O atoms bonding with Zn near and on the surface of said fine particles are liberated to leave numerous local zones in the surface, one type of which zone has an incomplete ZnO lattice with excessive $Zn^{++}$ and $Zn^{+}$ (positive ions) and the other has O atoms mostly remaining. For this reason, as illustrated in Fig. 1 showing a surface energy band structure of ZnO fine particle, in a zone A where a great number of O atoms are liberated (the left side surface zone of the particle 11), positive ions ($Zn^{++}$) are existing in excess on the surface to cause the energy band bended downward in the surface, at which position free electrons are accumulated and as a result a surface conductance is created. Also

protons $H^+$, generated by the decomposition of water, exist on the surface. Meanwhile in Fig. 1 Ev represents an energy level of valence band, Ef represents a Fermi level and Ec represents an energy level of the conduction band, respectively. On the other hand, in a zone B where O atoms are hardly liberated (the right side surface zone of the particle 11), $OH^-$ and $O^-$, which are generated by adsorption and decomposition of water and oxygen contained in air, are existing so that the energy band is bended upward in the surface resulting in an accumulation of holes on the surface. Thus in the surface of activated ZnO fine particle both free electron accumulation zone A and free hole accumulation zone B are distributing in a jumble.

Another important effect of activating ZnO by a heat treatment is that impurities contained in ZnO are accumulated in the surface layer of ZnO particle by said treatment to form impurity levels 12a and 12b within the forbidden band, which enables excitation of electrons and holes by the visible light irradiation leading to a capability of the photosynthesis.

The forbidden band gap (the difference between Ev and Ec) of ZnO crystal is 3.2 eV so that in order to create pairs of the free electron and hole by exciting electrons from the valence band to the conduction band over said forbidden band, light having an energy larger than 3.2 eV, i.e., UV rays having a shorter wavelength than 389 nm must be irradiated. In the present invention, however, as shown in the surface zone A of ZnO fine particle 11 in Fig. 1, the impurity levels 12a are lower than Fermi level Ef so that they act as donor levels, from which free electrons can be yielded by photoexciting electrons to the conduction band energy level, Ec.

While the impurity levels 12b as shown in the surface zone B of ZnO fine particle 11, are higher than Fermi level Ef so that they serves as acceptor levels, in which free electrons can be received by photoexciting electrons from the valence band, thus forming free holes in the valance band. Namely in the present invention through the heat treatment of ZnO the electron accumulation zone A and the hole accumulation zone B are formed on the surface of ZnO particle with its energy band bended near the surface and in addition to this impurity levels 12a and 12b, the former serving as a donor and the latter as an acceptor, are formed on the surface so that electrons can be excited by the visible light irradiation to yield free electrons and holes. Meanwhile although ZnO has a property of n-type semiconductor originating from a lattice defect and has a donor level 13 as illustrated by a dotted line 13 in Fig. 1, these factors contribute little to the surface conductance.

In the hole accumulation zone B on the surface of ZnO, the holes are transferred to adsorbing $H_2O$ molecules to generate $H^+$ as expressed by the following formula:

$$\tfrac{1}{2}H_2O + \oplus \rightarrow \tfrac{1}{4}O_2 + H^+ \qquad (2)$$

Protons $H^+$ thus generated transfer to the electron accumulation zone A due to a surface diffusion. While in the electron accumulation zone A on the ZnO surface, generated electrons and protons interact with $CO_2$ molecules to yield formic acid as expressed by the following formula:

$$CO_2 + 2H^+ + 2\ominus \rightarrow HCOOH \qquad (3)$$

It is considered that subsequent to this reaction transference of electrons and protons is successively repeated to yield HCOH, and further to form $CH_3OH$. Consequently the photosynthesis as expressed by the formula (1) is achieved as a whole. Separate from this the formation of formic acid may also be possible independent of the charge transference mechanism. What is significant here is that the ZnO surface is not homogeneous because of the zones existing in a jumble where reactions expressed by the equations (2) and (3) are to progress and that protons are readily surface-diffused. Therefore the reactivity of the formula (1) in the ZnO surface is considered to depend largely upon the activation treatment of the ZnO surface (fall of oxygen atoms from the lattice and the formation of impurity levels on the surface, both originating from the heat treatment). Furthermore such a situation is supposed to exist that decomposition of $CO_2$ and that of $H_2O$ are not independent of each other but cooperative to accelerate the reactions. The experimental results of IR absorption spectrum suggest that $CO_2$ and $H_2O$ are interacting each other on the ZnO surface and are susceptible to be a formate state readily by the light irradiation. Thus electrons photoexcited from the valence band to the acceptor impurity level, are consumed in the formation of minus ions on the ZnO surface or transfer into the conduction band by the photoexcitation as well as the tunnel mechanism so that the acceptor levels may be always unfilled. In such ways free holes to be used in the formula (2) can be successively supplied. On the other hand, although vacancy is also generated in the donor impurity levels by the photoexcitation they are easily filled by electrons coming from ions on the surface by the tunnel effect because the energy of the donor impurity levels are lower than that of Fermi level.

As stated above the present inventive photosynthesis of organic substances from $CO_2$ is different from Fujishima et al., in its essence. In Fujishima et al. method, free electrons and holes are generated by irradiating a higher energy (i.e., UV rays of $\lambda \leqq 390$ nm) than the forbidden band gap of ZnO, i.e., 3.2 eV ($\lambda$ = 389 nm) to excite electrons from the valence band directly to the conduction band (referred to as a bulk photoexcitation) and by high energy electrons and holes thus generated, the photosynthesis is promoted. In said method, however, powdery semiconductors are dispersed in water so that the surface of said semiconductor particles are almost covered with $H_2O$ molecules. As a result it becomes difficult for $CO_2$ molecules to reach the particle surfaces in enough amounts even if protons are yielded on the particle surfaces in accordance with the reaction expressed by the formula (2). Therefore it will be apparent that the reaction as expressed by the formula (3) occurs a few. On the contrary in the present inventive method the impurity levels on the surface, which are formed by the surface activation, are made to act as a donor or an acceptor and as a result free electrons and holes can be generated even by the visible light photoexcitation (referred to as a surface photoexcitation). Furthermore, since $CO_2$ and $H_2O$ are fed respectively as a gas, the amounts of said gases in the ZnO particle surface can be easily controlled to progress the reactions expressed by the formula (2) and (3) efficiently. As explained above the most important factors to photosynthesize organic substances from $CO_2$ by irradiating the visible light in the present invention are: the ratio of O relative to Zn on the surface of ZnO crystals varies in the surfacial direction; kinds of adsorbing charge are common such as $O_2^-$, $OH^-$ and $H^+$, etc.; and the surface band is locally bended upwardly (occurrence of hole accumulation layer) or downwardly (occurrence of electron accumulation layer) due to the surface impurity levels or the surface levels. In accordance with the present inventive method, electrons passing through the surface level, the process of hole excitation and the charge transference process can all be utilized efficiently and as a result both purposes of methanol synthesis by the sun light irradiation and of the stable use of ZnO crystals can be attained simultaneously. The principle of the present invention as stated above will be verified by the following experiments and measurements.

[Experiments]

Powdery ZnO specimen was supplied by ZCA, USA, of which surface area was 10 $m^2/g$. The specimen was fed in a glass cell 21 provided with a fused quartz window and was set in a vacuum apparatus as illustrated in Fig. 2. Valves $V_1$ and $V_2$ were opened to attain a high-vacuum and heat-degassing was carried out using an electric furnace 22, which was controlled by a thermostatic apparatus 22C, at 100°C for one hour and subsequently at 400°C for 4 hours to perform an activation heat treatment. A high-vacuum evacuation system was constructed by a rotary pump 23 and a molecular turbo pump 24 and can provide $10^{-8}$ Torr within a vacuum chamber 25 made of stainless steel. Various measurements under high-vacuum were conducted with the vacuum chamber 25 attached by, for example, a mass spectrometer 28 or an Auger electron spectrometer (not illustrated) and the obtained results were recorded by a recorder 28R. The vacuum degree within the chamber 25 at the time of measurement was detected by a vacuum gauge 25G. Ar sputter gun 29 was employed so as to clean the specimen surface by sputtering Ar thereto prior to Auger electron spectroscopic analysis. $CO_2$ and $H_2O$ were fed to the activated ZnO powder via valves $V_4$ and $V_5$ and feed pressures were adjusted by operating said valves while observing a pressure gauge 26. Said gases were fed with the valve $V_1$ closed while valves $V_2$ and $V_3$ were opened and the gas systems were exhausted by means of a rotary pump 27.

Infrared rays (IR) absorption test was carried out using Perkin-Elmer 1800 FTIR apparatus (not shown) at a room temperature by means of a quartz window or KBr window in such a manner that ZnO powder was allowed to adhere to said window to measure the transmitted infrared rays. Analysis of photosynthesis products was done by means of a quadruple mass spectrometer (Balzers OMG-112A) 28. The Specimens were illuminated by a Xe lamp 19 of 75W serving as a light source of the surface photosynthesis, with or without a filter 18 which is to cut UV light shorter than 390 nm. Otherwise the specimen cells were so set outdoors as to be irradiated by the sun light in a right angle to carry out the photosynthesis.

[Measurement Results]

First, in Fig. 3 there is shown IR absorption spectrum of adsorbed $CO_2$, which is considered to contribute to the photosynthesis. IR absorptions of antisymmetric stretching vibration modes of the adsorbed $CO_2$ molecules are shown as curves 3a and 3b, the former being obtained by $CO_2$ (150 Torr) supply alone and the latter by $CO_2$ (150 torr) and $H_2O$ (20 Torr) supply, both subsequent to the activation of ZnO fine particles at 400°C. For convenience sake, the curve 3a is shifted upward because curves 3a and 3b mostly overlap each other in the common coordinates. Meanwhile a thick line in the figure shows IR

absorption of ZnO free from gas supply, which agrees with the characteristics of the quartz window itself provided to the cell 21 because the characteristics of ZnO happens to be flat within this measurement range. The spectrums were obtained by IR transmittance measurement with ZnO fine particles in the cell 21 adhered to the wall of the quartz window.

Frequency of the stretching vibration mode of the free $CO_2$ molecules is known to be 2349 $cm^{-1}$ and each of curves 3a and 3b has IR absorption spectrum structure in which band becomes wide around the frequency of free $CO_2$ molecules at the center. This structure of the absorption spectrum is observed very faintly in curve 3a, without $H_2O$ supply, whereas considerably solid in curve 3b, with $H_2O$ supply. This means that molecular $CO_2$ hardly adsorbs to the surface of ZnO fine particles when OH groups are absent therein. It has been known that chemically adsorbed $CO_2$ on the ZnO fine particles has IR absorption at 1490 - 1640 $cm^{-1}$, which can be regarded as a carboxylate state, and that the amount of adsorbed $CO_2$ is approx. 1% relative to the site number of ZnO surface. This adsorption is assumed to correspond to the absorption structure of the curve 3a, so that the solid absorption structure of $CO_2$ as noted in curve 3b can be considered to originate from molecules generated by an interaction of $CO_2$ molecules with OH groups of $H_2O$ adsorbing to the surface of ZnO fine particles. This type of adsorption will be desorbed in its 60% by an evacuation at room temperatures.

The absorption structure of stretching vibration mode of the curve 3b was examined in detail by measuring IR absorption spectrum by means of KBr window which has a flat characteristic within the measurement range, the result of which are shown in Fig. 4. Relative to the stretching vibration frequency of free $CO_2$ molecules, the polarization to plus of $CO_2$ molecules will result in a higher stretching vibration frequency, whereas polarization to minus will result in a lower frequency. Many absorption spectrums 4a through 4g existing near the stretching vibration frequency of free $CO_2$ molecules, 2349 $cm^{-1}$, suggest that $CO_2$ molecules are existing in various polarized states as well as in bonding states with OH groups on the surface of the ZnO fine particle and such $CO_2$ molecules in various states are considered to be related to various reactions in the course of formic acid formation to methanol formation.

Next the light irradiation effect on these adsorbing species will be explained. In Figs. 5 and 6A, there are shown changes of IR absorption spectrum with the irradiation time as curves 5a, 5b and 5c when the ZnO fine particles are supplied with $CO_2$ (150 Torr) and $H_2O$ (20 Torr) and are irradiated by the sun light (Fig. 5) and by a Xe lamp (75 W without filter, i.e., the illumination contains UV rays having a higher energy than the forbidden band gap of ZnO) (Fig. 6A). For convenience sake, these curves are also shifted upward in turn to avoid overlapping. In Fig. 5 of the sun light irradiation, curves 5a, 5b and 5c respectively represent IR absorption spectrum of: before irradiation, one hour after irradiation, and two hours after irradiation, while in Fig. 6A, curves 6a, 6b and 6c respectively represent that of: before irradiation, 10 minutes after irradiation, and 20 minutes after irradiation. Only when the ZnO particles are irradiated for more than one hour by the sun light or for more than 10 minutes by the Xe lamp, a decrease in IR absorption spectrum of $CO_2$ adsorbing on ZnO surface can be observed. In the present experiment it is assumed that regardless of the presence of $CO_2$ gas of 150 Torr in the gas phase of the measuring cell, the adsorption sites of $CO_2$ (an appropriate interaction state with OH groups) were lost or ZnO surface was occupied by other molecules. Namely the results suggest that the adsorbing $CO_2$ molecules were consumed by the photosynthesis.

Curves 6a and 6c in Fig. 6A were examined in their IR absorption at the shorter wavelength side, the results of which are shown in Fig. 6B. Originating from the Xe lamp irradiation for 20 minutes, the absorption of C-H vibration is observed near 2900 $cm^{-1}$, which is indicated by arrows. This apparently means that an organic substance was yielded on the ZnO surface by the photosynthesis. Additionally, in Figs. 7A and 7B IR absorption spectrums obtained before the sun light irradiation and after irradiation for 2 hours, were respectively shown, which were measured by means of a KBr window and with a supply of $CO_2$ (150 Torr) and $H_2O$ (20 Torr). As indicated by an arrow, after the irradiation for 2 hours the amount of adsorbing $CO_2$ molecules became almost half and absorption spectrums possibly originating from methanol molecules (near 2900 $cm^{-1}$ and near 1100 $cm^{-1}$) as well as formate (near 1350 $cm^{-1}$) were observed. From the results it is assumed with a high reliability that the employment of ZnO, which is surface activated by the present inventive method, will lead to a formation of organic substances including methanol through photosynthesis from $CO_2$ and $H_2O$ even by an irradiation of sun light which little includes UV rays.

Next such process will be verified that by a visible light having a lower energy than the forbidden band gap of ZnO, i.e., $h\nu$ = 3.2 eV, free electrons and holes are surely excited on the ZnO surface and that they are actually utilized in the reactions. For this purpose the surface conductance of ZnO single crystal was measured. Fig. 8 shows an influence of gas adsorption upon the surface conductance of a transparent, needle single crystal of the activated ZnO (1010) in a size of 3 mm$\phi$ × 17 mm. ZnO single crystal was activated at 800°C for 10 minutes and then Au was vacuum deposited thereto to form electrodes at both ends on its surface. The resultant was taken out in the air within a clean room (class 1000) and was set

within the apparatus as illustrated in Fig. 2 followed by evacuation. Then $CO_2$ was fed slowly into the gas feed apparatus and while elevating the pressure p the change of surface resistance $\delta R$ was measured with time. At the time of 450-second-elapse a mixture gas consisting of $CO_2$ and $H_2O$ was fed thereto to carry out measurements in the same manner. Thus the ZnO surface to be measured was partially covered at first with OH groups. The results of Fig. 8 suggest that the reduction of the surface conductance (an increase in resistance $\delta R$) is further promoted by adsorption of the mixture gas ($CO_2$ + $H_2O$) and that free electrons transfer to $CO_2$ molecules (formation of $CO_2{}^{-\delta}$).

Next the surface photocurrent excited by the monochromatized light irradiation was measured using the same specimens and the obtained results are shown. The surface photoconductance was measured at a room temperature (22° C) by irradiating a monochromatized light with a wavelength of $\lambda$ = 397 nm (3.1 eV) which was obtained by a Xe lamp as a light source with an interference filter of $\delta\lambda$ = 10 nm width. The single crystals of ZnO applied by a constant voltage of 0.8 V were respectively retained under vacuum, under $CO_2$ atmosphere of 150 Torr, and under an atmosphere of $CO_2$ of 150 Torr and $H_2O$ of 20 Torr to measure respective photoexcited currents generated by the light irradiation, the results of which are shown in Figs. 9A, 9B and 9C respectively. From the results, excitation (or relaxation) of the photocurrent was found to be considered of a rapid component (response within 5 seconds from the start or finish of the light irradiation) and a slow component (response after 5 seconds or more from the start of cut off of the light irradiation) and to be dependent largely upon the adsorbing species (reactive species) present on the ZnO surface.

With the irradiation of monochromatized light of $\lambda$ = 500 nm (2.5 eV) measurements were carried out in the same way as in Figs. 9A, 9B and 9C, the results of which are shown in Figs. 10A, 10B and 10C respectively. From the results the excitation of photocurrent was also found to be dependent largely upon the gas atmosphere but the currents themselves were found respectively to become about 1/4 of the irradiation wavelength of 397 nm, i.e., Figs. 9A, 9B and 9C. Meanwhile the phenomenon where the photoexcited current did not get zero even by cutting off the light after irradiation, was much prominent under a higher vacuum and this is considered that due to the light irradiation charges (electrons, holes and adsorbing ions) are accumulated on the ZnO surface resulting in a change of the surface band bending. In the same way experiments were conducted varying the incident wavelength upto 650 and the results are summarized in Fig. 11. The photocurrents in these experiments are the rapid, excited current after 5 seconds from the irradiation. It can be understood from the results that: the photoexcited current is small when $\lambda$ = 500 nm or higher and that of under ($CO_2$ + $H_2O$) gas atmosphere (i.e., curve 11c) is smaller than that under vacuum (curve 11a), and that the difference therebetween is originating from consumption of the excited electrons and holes by the surface photoreaction. The results of Fig. 11 mean that the visible light not higher than $\lambda$ = 500 nm is utilized in the reaction of $CO_2$ and $H_2O$.

So as to be capable of progressing reaction between $CO_2$ and $H_2O$ on the ZnO surface by the visible light excitation, the surface impurity levels 12a and 12b must be present as explained in Fig. 1. Results of the surface element analysis of the ZnO surface by means of an Auger electron spectrometer are summarized in Fig. 12. There are shown ratios (%) of the major impurities detected on the ZnO surface of such specimens as: ZnO single crystals without activation, ZnO powder prior to activation, ZnO powder after activation, and ZnO powder after photoreaction by a Xe lamp. It was found that impurities S and P were accumulated on the surfaces of ZnO fine particles by the heat treatment at 400° C. Meanwhile in ZnO fine particles impurity Si is always present and the surface concentration of said impurity was found to be decreased slightly by the heat treatment.

Taking the above described results into consideration, ZnO fine particles were exposed to the sun light in winter (December) and in summer (June) and thus generated products were subjected to a mass spectrometric measurement, the results of which are respectively shown in Figs. 13 and 14. In this experiment the activated ZnO fine particles were fed by $CO_2$ (150 Torr) and $H_2O$ (20 Torr) and thus treated cell 21 (Fig. 2) was taken to outdoors to be irradiated by the sun light at right angles for about 3 hours. At this time the back surface of the cell 21 was also lighted by means of foil A1. Thereafter the gas composition was measured by a mass spectrometer. The ambient temperature in December test (Fig. 13) was -2~0° C while that in June was 18~22° C. The composition analysis of the gas in cell 21 was carried out in the following procedure: (a) gas phase constituent analysis; (b) analysis of molecules weakly adsorbing to ZnO by means of an evacuation; (c) by conducting RF current (125 kHz, 1.2A) to the coil attached to the cell surface, ZnO fine particles are allowed to be heated from 20° C to approx. 45° C and thus desorbed molecules are subjected to a mass spectrometer. The analysis results obtained by (a) and (b) are not shown here and the results in Figs. 13 and 14 are obtained by analysis (c). Kinds of products obtained by analyses (b) and (c) were the same but the amount of organic products in (c) was several times greater than (b). In analysis (a) the presence of $O_2$ molecules was often confirmed. In Figs. 13 and 14 there

are shown major kinds of molecules desorbed by the temperature rise in the analysis (c). The desorbed amounts of $CO_2$ (M/e = 44) and $H_2O$ (18), which are the reactive species, reached several times greater than other desorbed molecules, so that they are not shown here. Meanwhile the following results of mass analysis are the actual measured values, namely no correction is made relative to differences in ionization probability as well as in fragment ratio depending on kinds of molecules, so that the relative intensity ratio has only a qualitative meaning. The characteristic of Fig. 14, summer test result, is the detection of the molecule kind of M/e = 43, which can be considered as $H_nCCO$ ($C_2$ - O). For the sake of comparison, analysis results of products obtained by irradiation of white Xe lamp (without filter) of 75 W for 20 minutes, in accordance with analysis (c), will be shown in Fig. 15. As is clear from the figure methanol formation was a little and M/e = 43 ($C_2$ - O) was the major productant molecule. Consequently, to photosynthesize methanol, the present inventive method by the visible light irradiation is advantageous as is apparent from Figs. 13 and 14, where the major component generated is methanol.

Nextly another experimental results of photoreaction product analysis will be described below. The activated ZnO fine particles were fed with $CO_2$ (150 Torr) and $H_2O$ (20 Torr) and after evacuating gas phase for 5 minutes they were irradiated by a white Xe lamp (without filter, i.e., including UV rays) to measure desorbed gas directly by means of a mass spectrometer, the results of which are shown in Fig. 16. From the figure it becomes apparent that an increase or occurrence of ion current was caused by the light irradiation and also desorptions of M/e = 31 ($CH_3OH$), 29 ($C_2$), 43 ($C_2$ - O), and 32 ($O_2$) were observed. An irradiation test was carried out in the same manner using a filter so as to remove UV rays (E > 3.2 eV) from the Xe lamp illumination and thus obtained products were shown in Fig. 17. Fig. 16 shows that not only methanol (M/e = 31) but also other various kinds of molecules (M/e = 29, 32, 43, etc.) were yielded in almost equal amounts by the irradiation of Xe lamp illumination including UV rays. Whereas Fig. 17 shows that the major product was methanol by the irradiation of Xe lamp illumination free from UV rays. Namely it will be clearly understood that photosynthesis by the visible light can produce methanol efficiently.

Lastly the surface photoreaction will further be proved in Fig. 18. The experiment was conducted in such a manner that: ZnO fine particles were fed with $CO_2$ (150 Torr) and $H_2O$ (20 Torr) and after evacuating gas phase upto 0.5 Torr changes of gas pressure within a closed-type cyclic reactor were measured by means of a Pirani vacuum gauge. The pressure reduction with time was observed because of the successive adsorption of the gas phase onto ZnO but when the visible light ($\lambda$ = 370 - 670 nm) was irradiated the pressure reduction was found further accelerated after approx. 30 seconds from the irradiation due to the surface photoreaction. It can be said that since 4.5 g of ZnO powder were used in this experiment the accumulation of reaction products on the ZnO surface was effectively performed.

[Discussion]

(a) Excitations of free electrons and holes due to the visible light irradiation

Utilization of UV rays so as to excite electrons and holes via bulk photoexcitation is quite apart from the present inventive purpose, utilization of the sun light. Further the former has been known to decompose the ZnO crystals because of its process of yielding $O^-$, $Zn^+$ ions by photoexcitation. Also an Auger electron spectroscopic analysis has made it clear that the photoreaction of $CO_2$ by UV rays promotes the accumulation of graphite on the ZnO surface. In the photoreaction of ZnO single crystals, this effect is particularly prominent so that the whole surface will be covered with graphite to lose the RHEED pattern of ZnO. This fact will become a serious obstacle to perform a stable surface photoreaction for a long period. Whereas according to the present inventive method, in which the production mechanism of free electrons and holes by the visible light via surface impurity levels (the surface photoexcitation) is utilized, neither the decomposition of ZnO nor accumulation of graphite on the surface occurs so that the photoreaction can be continued stably.

ZnO fine particles actually employed in experiments unavoidably contained impurities of Si, S and P, among which S and P were confirmed by AES analysis to be accumulated in the surface by the activation heat treatment of the specimen (Fig. 12). Photoexcited current illustrated in Figs. 9A, 9B and 9C proves that the surface photoexcitation mechanism via surface impurities as described above, is functioning especially at $\lambda \leq 500$ nm (2.5 eV). These impurity levels contribute to the band bending in the surface at the same time to allow electrons or holes concentrated on the surface resulting in an appearance of the surface conductance. It is essential for the surface chemical reactions that free electrons or holes on the surface can transfer to the adsorbing $CO_2$, OH, $H_2O$ or HCOOH, which has been confirmed by the present inventors using ESR method, namely the charge transfer to $CO_2$ molecules or $H_2O$ molecules adsorbing on the ZnO surface can readily occur at or below the room temperature. (M. Watanabe and T. Ito, Jap. J. Appl. Phys.,

19, 1853 (1980); 19, 1863 (1980); Proc. 4th Int. Conf. Solid Surfaces, Cannes (1980), p440.) As described above the hatched region in Fig. 11 is proved to correspond to amounts of free electrons and holes consumed in the surface reaction.

(b) State and reactivity of $CO_2$, $H_2O$ on the ZnO surface

Chemically adsorbing $CO_2$ molecules or formates on the ZnO surface have been known to play an important role in a catalytic reaction at 200°C or higher. $CO_2$ molecules, which play the leading part in the present inventive photosynthesis, are different from the chemically adsorbing type and are those adsorbing weakly with or near OH groups on the surface as is observed in IR absorption spectrums in Fig. 3 and Figs. 7A and 7B and said molecules are considered to be ionized (polarized) weakly ($CO_2^{+\delta}$, $CO_2^{-\delta}$). As shown in Fig. 7A IR absorption spectrums of OH groups at 3200 - 3700 $cm^{-1}$ and near 1600 $cm^{-1}$ do not change largely by $CO_2$ supply, from which it can be said that the presence of OH groups on the surface is essential for adsorption of $CO_2$ ions onto ZnO but that the interaction between OH groups and $CO_2$ molecules is weak. Due to the photoreaction by the irradiation, however, absorption at 3700 $cm^{-1}$ disappears mostly (Fig. 7B) so that the adsorbing $CO_2$ molecules are considered to react easily with OH groups through the charge transfer mechanism to form formate or formic acid first. As is clear from Figs. 5 and 6A the total amount of adsorbing $CO_2$ molecules is decreased by the irradiation and especially $CO_2$ having a low wave number of 2300 $cm^{-1}$ disappears entirely. This is understood that the low wave number $CO_2$, which is intensively interacting with OH group on the surface, is related to photosynthesis, while other $CO_2$ with a high wave number transfers to said low wave number molecule type. Meanwhile formates, methanol molecules, etc., which are yielded by photosynthesis, are mostly locating on the ZnO surface to occupy the adsorption sites of $CO_2$.

(c) Photoreaction products and mechanism

The most significant results concerning products are that the major product by the visible light irradiation is $CH_3OH$ as shown in Figs. 13, 14 and 17 accompanying $O_2$ as shown in Fig. 17, whereas products by Xe lamp irradiation including intense UV rays are $C_2$ - O and $CH_4$ molecules as shown in Fig. 15. Consequently products obtained by the surface photoexciting mechanism by the visible light can be said, in accordance with the formula (1), $CH_3OH$ and $O_2$. While products by the bulk photoexcitation mechanism by UV rays irradiation are hydrocarbons and $C_2$ - O. In the latter case graphites are accumulated on the ZnO surface, which results in a rapid fall of the photoreactivity of ZnO.

Amounts of organic products existing on the surface can be roughly estimated from the results of mass analysis of Fig. 13. Relative to the amount of $CO_2$ molecules adsorbing the surface, amounts of $CH_3OH$ and $CH_4$ produced by the sun light irradiation is approx. 5%. Durability of photoreaction is regarded as excellent remarkably in case of the sun light irradiation (visible light irradiation). Even when the product analysis by the sun light irradiation and gas phase evacuation was repeated four times, the product amount did not vary extremely. The upper limit of the photoconversion efficiency is related to the reverse reaction, i.e., photodecomposition susceptibility of methanol. Then to the activated ZnO surface a mixture gas of 80% methanol and 20% $H_2O$ was supplied and after a short evacuation a Xe lamp illumination was irradiated thereto to measure the decomposition. Decomposition upto $CO_2$ was seldomly observed but the formation of $CH_4$ was found.

Next the photoreaction mechanism of synthesizing $CH_3OH$ from $CO_2$ and $H_2O$ molecules will be discussed here. IR absorption spectrums in Figs. 7A and 7B suggest that $CO_2$ molecules are bonding with ZnO via OH groups on the surface before irradiation and that formates (1370 $cm^{-1}$)

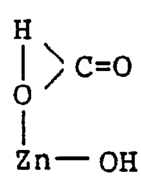

or bidentate formate

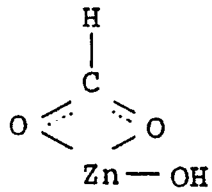

are yielded by irradiation. Presumably these formates are intermediates of the present photoreaction. The reduction-decomposition reactions of $CO_2$ and $H_2O$ seem to be cooperative to promote each other through the interaction within the adsorbing layer. Actually decomposition to OH will not be observed when $H_2O$ on the ZnO surface is irradiated by the visible light. It is apparent from the surface photoconductance tests that the photoreduction of $CO_2$ and $H_2O$ molecules is a reaction caused by the transference of electrons and holes on the ZnO surface. The methanol synthesis by the sun light irradiation is advantageously carried out at low temperatures (winter season) (Figs. 13 and 14). As discussed by Fujishima et al., in methanol formation the electron-proton transference mechanism

$HCOOH + 2H^{+} + 2 \ominus \rightarrow HCHO + H_2O,$
$HCHO + 2H^{+} + 2 \ominus \rightarrow HC_3OH$

is considered to have reactivity superior than the thermal catalytic reaction. To promote the former reaction protons yielded in the surface of the hole accumulation layer must be diffused to the surface of the electron accumulation layer. Thus it may be concluded that in the surface of the ZnO fine particles activated by the heat treatment, both kinds of accumulation layers are appropriately distributing.

As explained hereabove the results obtained by experiments are summarized as follows:

(1) By the activation heat treatment at 400°C, the surface conductance appears in the surface of ZnO fine particles, in which electron accumulation layer as well as hole accumulation layer is formed locally.

(2) By the activation heat treatment, S and P impurities are allowed to diffuse from inside to concentrate in the ZnO surface, and as a result the surface impurity levels are produced.

(3) The visible light irradiation causes excitation of electrons and holes via the surface impurity levels and thus the surface conductance is further increased.

(4) The charge transference from the surface accumulation layer to the adsorbing molecules is relatively easy, which may result in that ZnO is active in the methanol synthesis.

(5) $CO_2$ molecules are weakly adsorbing to the ZnO surface via OH groups and the adsorbing amount thereof is close to the number of OH group.

(6) The photoreduction of $CO_2$ and $H_2O$ molecules on the ZnO surface progresses in the adsorbing state respectively serving as a promotor to each other.

(7) The conversion ratio in methanol synthesis from $CO_2$ and $H_2O$ by the visible light irradiation is about 5% relative to $CO_2$ amount adsorbing the surface.

[Embodiment]

In Fig. 19 there is shown a conversion apparatus from $CO_2$ gas to organic substances in accordance with the present invention. The reaction vessel 21 is constructed in a disc shape made of, for example, glass or fused quartz so as to get a wider light receiving surface, inside of which vessel is filled with ZnO powder. The particle size of said ZnO fine particle is about 100 nm in diameter. The reaction vessel 21 is set in an electric furnace 22 by adjusting the position of the furnace upward or downward to carry out the heating of said fine particles contained in the vessel 21. Gases in the vessel 21 can be evacuated by a rotary pump 32 via an evacuation pipe 45 and a valve 31. $CO_2$ gas from the $CO_2$ gas source 33 is supplied to a pipe 35 by opening a valve 34, while nitrogen gas from the nitrogen gas source 36 is also supplied to the pipe 35 by opening a valve 37. The pipe 35 is optionally attached by a flowmeter 38. The gas through the pipe 35 is dividedly supplied to valves 39 and 41 and the gas from the valve 41 is discharged into water contained in a water vessel 42, from which gas containing steam is supplied via valve 43 to a feed pipe 44. Gas via valve 39 is also supplied to the feed pipe 44. Through the pipe 44 gas is supplied to the reaction vessel 21 and passing through between ZnO fine particles the gas is evacuated to the pipe 45. Thus evacuated gas from the evacuation pipe 45 is fed to a thermotrap 46 to collect products. While the gas from the thermotrap 46 through an evacuation pipe 47 is discharged outside from a valve 48 or is recycled to the

pipe 35 via a circulator 49. One surface of the reaction vessel 21 is directly irradiated by the sun light 51 while the back surface is irradiated by means of a reflection mirror 52.

First the reaction vessel 21 is arranged in the electric furnace 22 and while the inside of the vessel 21 is evacuated to vacuum by the rotary pump 32 with the valve 31 opened, heating is done at the temperature from 100 to 400°C for 5 hours to subject the ZnO powder to the activation treatment. The heating treatment at about 400°C or higher should be continued for at least one hour but need not be longer than 10 hours. The heating temperature should not exceed 800°C so as not to damage the ZnO particles. High temperature vacuum evacution will allow $H_2O$, $O_2$ and other impurities, which are present on the surface of the ZnO fine particles, desorbed from the ZnO and simultaneously will allow excess $Zn^{++}$ yielded on the surface. At the time of this activation treatment valves 37 and 39 may be opened to supply $N_2$ gas.

Next the electric furnace 22 is removed and with the valve 31 closed, while the valves 34, 41 and 43 opened, $CO_2$ gas is supplied together with steam under about ambient pressure and the circulator 49 is operated with the reaction vessel 21 irradiated by the sun light 51.

$CO_2$ gas is converted within the reaction vessel 21 to organic substances like ethane, methanol, paraffin and olefin through the photocatalytic reaction, and the productant organic substances are collected to the thermotrap 46. To discharge thus produced organic substances from the vessel 21, valves 34, 41 and 43 are closed with valves 37 and 39 opened and at the same time the vessel 21 is heated by the electric furnace 22 and thus the products are fed to the thermotrap 47 using nitrogen gas as a carrier. When such a purge of products is conducted periodically the conversion of $CO_2$ to the organic substances can be performed efficiently. When the moisture is abundant even at the time of $CO_2$ supply the valve 34 side may be circulated. The productant organic substances can be separated to the respective constituents by heating the thermotrap 46 (or by cooling it) through the distillation separation process.

By the way, when the visible light was irradiated continuously to the ZnO fine particles according to the present inventive photosynthesis, yield of ethanol began to decrease after a certain elapse of time from the irradiation and finally to almost zero. Then the visible light irradiation was stopped for certain period and thereafter was started again to find the ethanol formation started. Further experiments were conducted and a repetition of the visible light irradiation for 5 minutes followed by a pause for 5 minutes, for example, was found to result in an increase of ethanol yield. Consequently it is recommended in the practical photosynthesis of ethanol that the light irradiation is intermittently conducted with an appropriate cycle as well as duty ratio.

This phenomenon will be explained as follows. When the amount of light irradiated per unit time becomes large as compared with the reaction rates of formulas (2) and (3), plus ions or minus ions are excessively accumulated respectively in the free electron accumulation zone A or free hole accumulation zone B, which will cause the bending of energy band in the surface extremely inclined. As a result electrons and holes are hardly photoexcited to stop the ethanol formation. But when the light irradiation is stopped, excess ions of plus or minus respectively in the accumulation zone A or B are surface-diffused to neutralize each other in an almost equilibrium state so that the energy band bending will get restored as the initial state. Thus free electrons and free holes become capable of being photoexcited readily.

As described above in accordance with the present inventive method the photosynthesis by the visible light using $CO_2$ and $H_2O$ can produce organic substances safely and economically and is practically applicable to industrial purposes.

Meanwhile the present invention has been explained on the photosynthesis using ZnO powder and using other metal oxides or a mixture of metal oxides which are heat activated, organic substances can also be photosynthesized from $CO_2$ and $H_2O$ by the visible light in the same way.

## Claims

1. Method for photosynthesis of organic substances from carbon dioxide and water comprising the steps of:
   a) heating a metal oxide to activate the surface thereof;
   b) supplying $CO_2$ gas and $H_2O$ gas to said metal oxide whose surface is activated; and
   c) irradiating visible light to said metal oxide to produce organic substances from said $CO_2$ gas and said $H_2O$ gas through a photochemical reaction.

2. The method according to claim 1, wherein said heat treatment includes a heating at a temperature not lower than 400°C for 4 hours or more.

3. The method according to claim 1 or 2, wherein said heat treatment is conducted under vacuum.

**4.** The method according to any of claims 1 to 3, wherein said metal oxide includes ZnO.

**5.** The method according to any of claims 1 to 4, wherein said visible light is sun light.

**6.** The method according to any of claims 1 to 5, wherein among said organic substances the major substance is methanol.

**7.** The method according to any of claims 1 to 6, wherein said steps further comprise a step of heating said metal oxide so as to dissociate said organic substances produced, from said metal oxide.

**8.** The method according to claim 7, wherein said steps further comprise a step of supplying nitrogen gas, which serves as a carrier, and removing said dissociated organic substances from the reaction system.

**9.** The method according to any of claims 1 to 8, wherein said metal oxide is in a form of powder.

**10.** The method according to claim 9, wherein said metal oxide is powdery ZnO.

**11.** The method according to any of claims 1 to 10, wherein said visible light irradiation is conducted intermittently.

FIG. 1

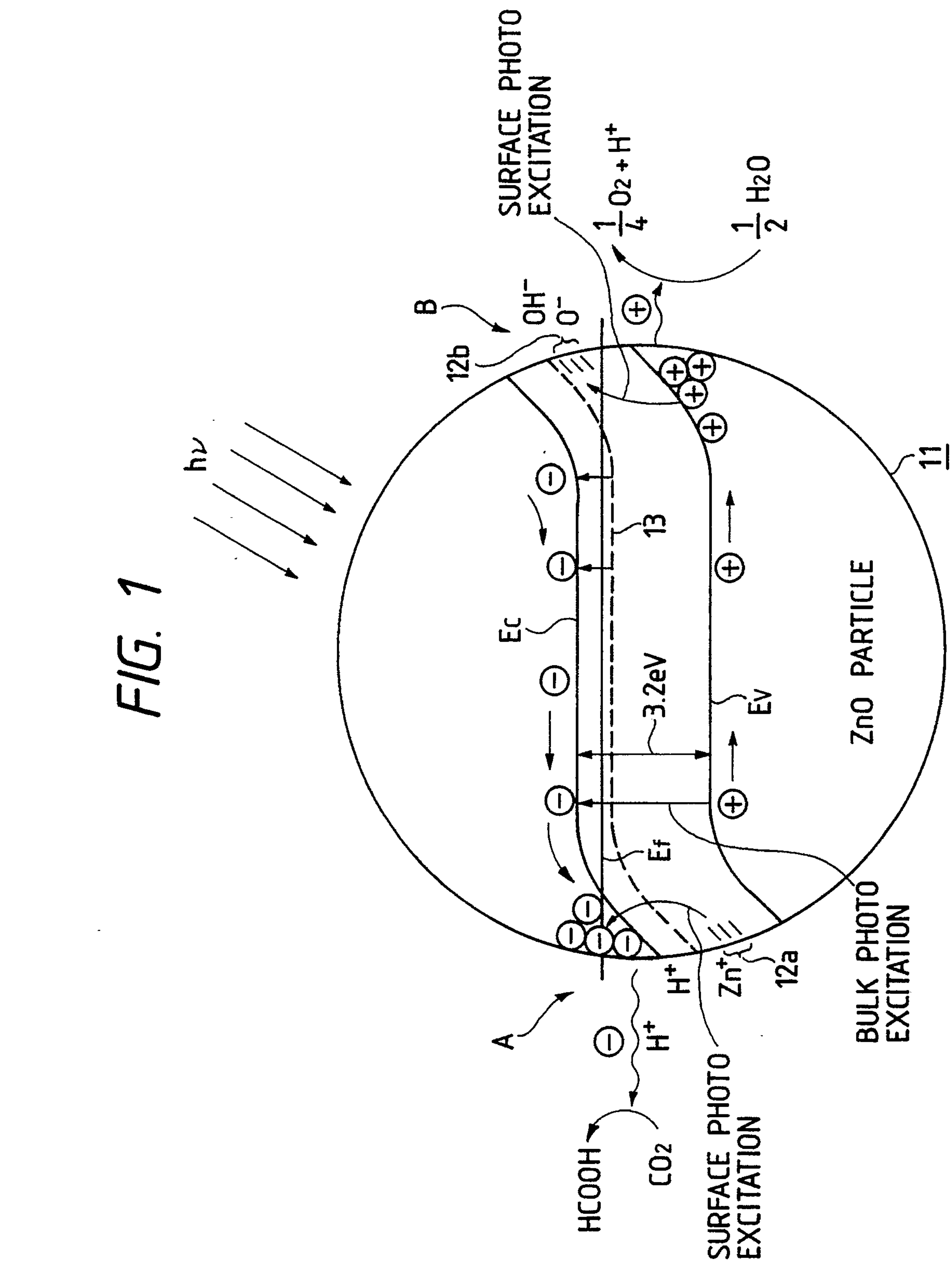
SURFACE PHOTO EXCITATION
$\frac{1}{4} O_2 + H^+$
$\frac{1}{2} H_2O$
B
$OH^-$
$O^-$
12b
hν
Ec
13
3.2eV
Ev
ZnO PARTICLE
11
Ef
A
$H^+$
$H^+$
$Zn^+$
12a
BULK PHOTO EXCITATION
HCOOH
$CO_2$
SURFACE PHOTO EXCITATION

FIG. 2

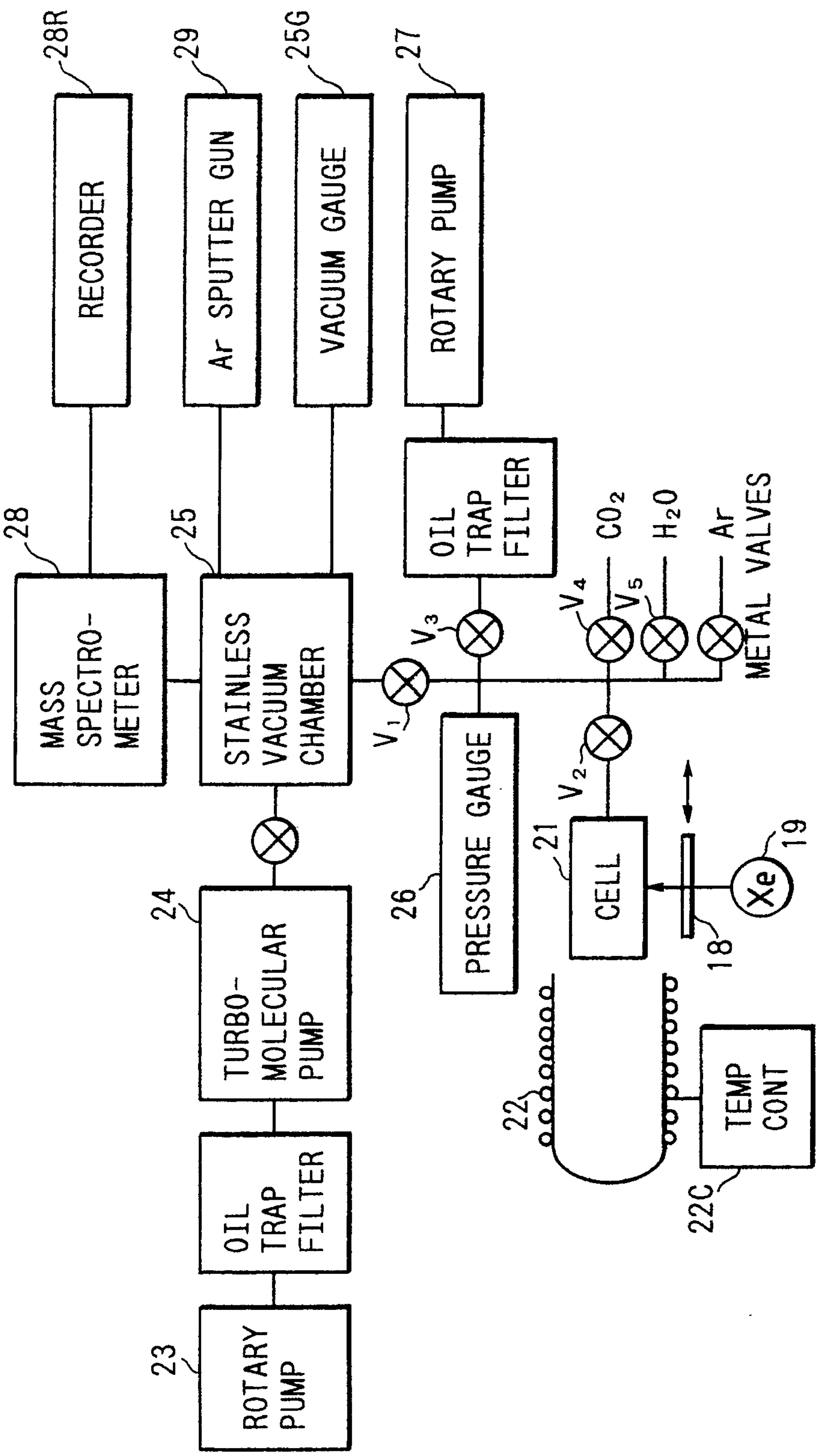
RECORDER
28R
Ar SPUTTER GUN
29
VACUUM GAUGE
25G
ROTARY PUMP
27
MASS SPECTRO-METER
28
STAINLESS VACUUM CHAMBER
25
OIL TRAP FILTER
$V_1$
$V_2$
$V_3$
$V_4$
$V_5$
$CO_2$
$H_2O$
Ar
METAL VALVES
PRESSURE GAUGE
26
CELL
21
18
Xe
19
TURBO-MOLECULAR PUMP
24
OIL TRAP FILTER
ROTARY PUMP
23
22
TEMP CONT
22C

FIG. 3

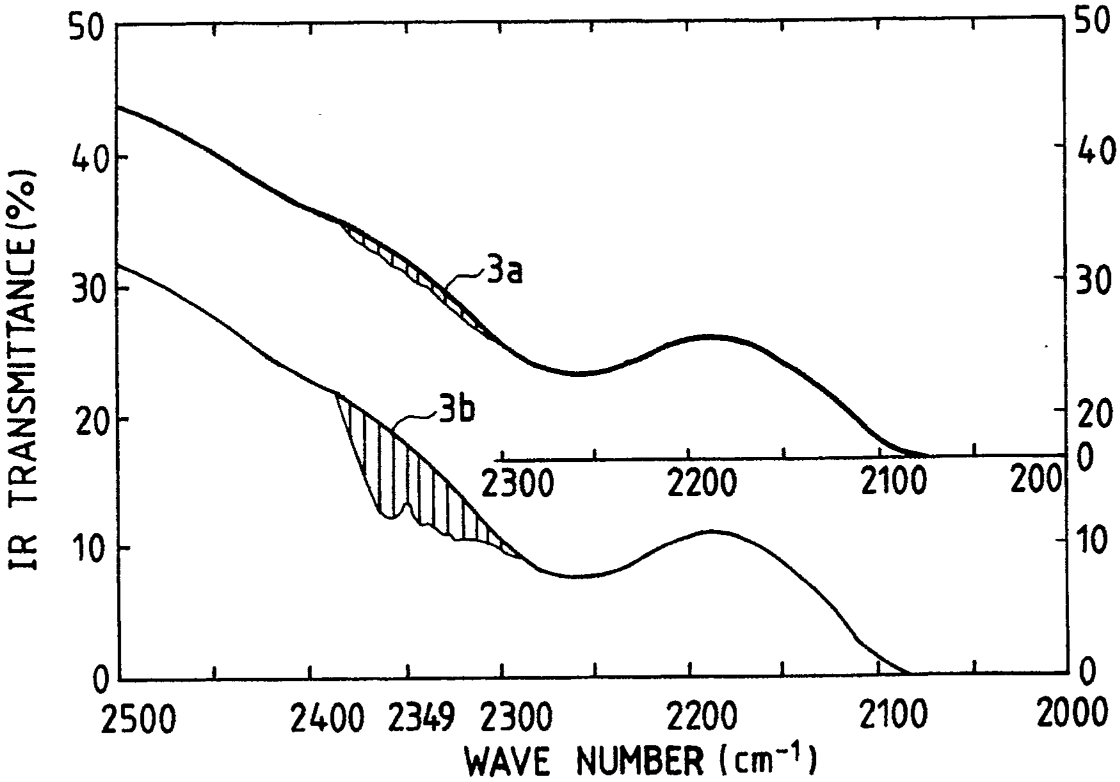
50
40
30
20
10
0
50
40
30
20
0
10
0
3a
3b
2300
2200
2100
200
2500
2400
2349
2300
2200
2100
2000
IR TRANSMITTANCE (%)
WAVE NUMBER (cm⁻¹)

FIG. 5

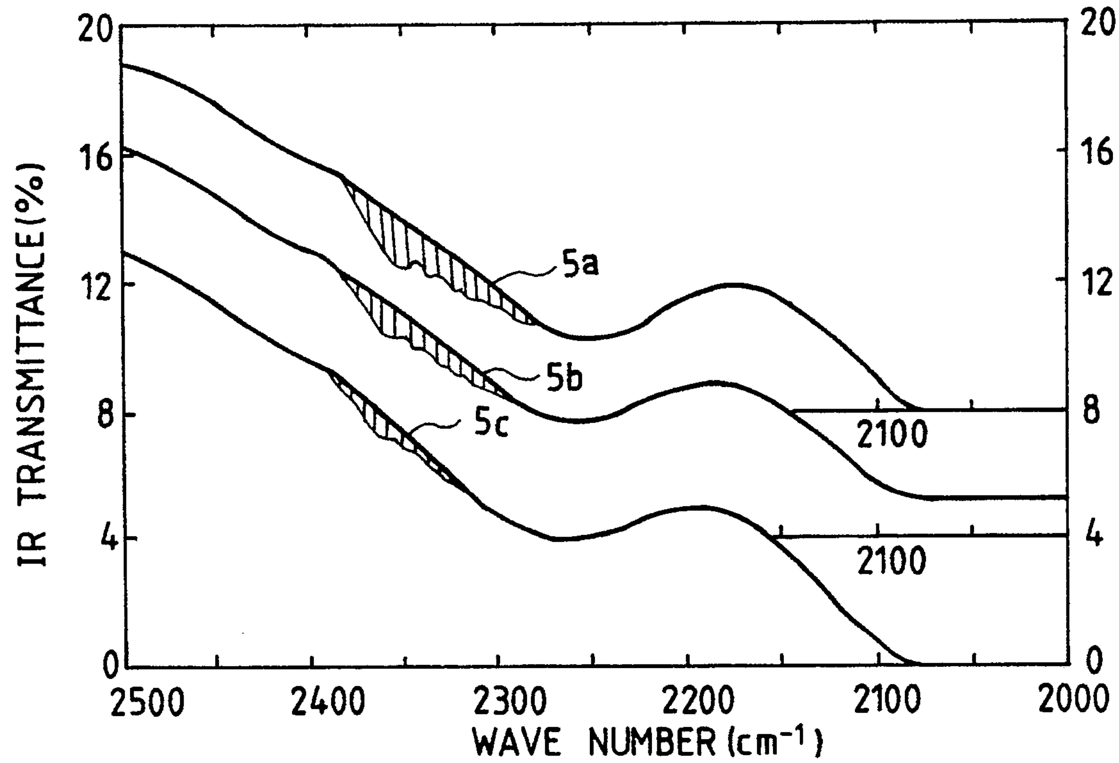
20
16
12
8
4
0
5a
5b
5c
2100
2100
2500
2400
2300
2200
2100
2000
IR TRANSMITTANCE (%)
WAVE NUMBER (cm⁻¹)

FIG. 4

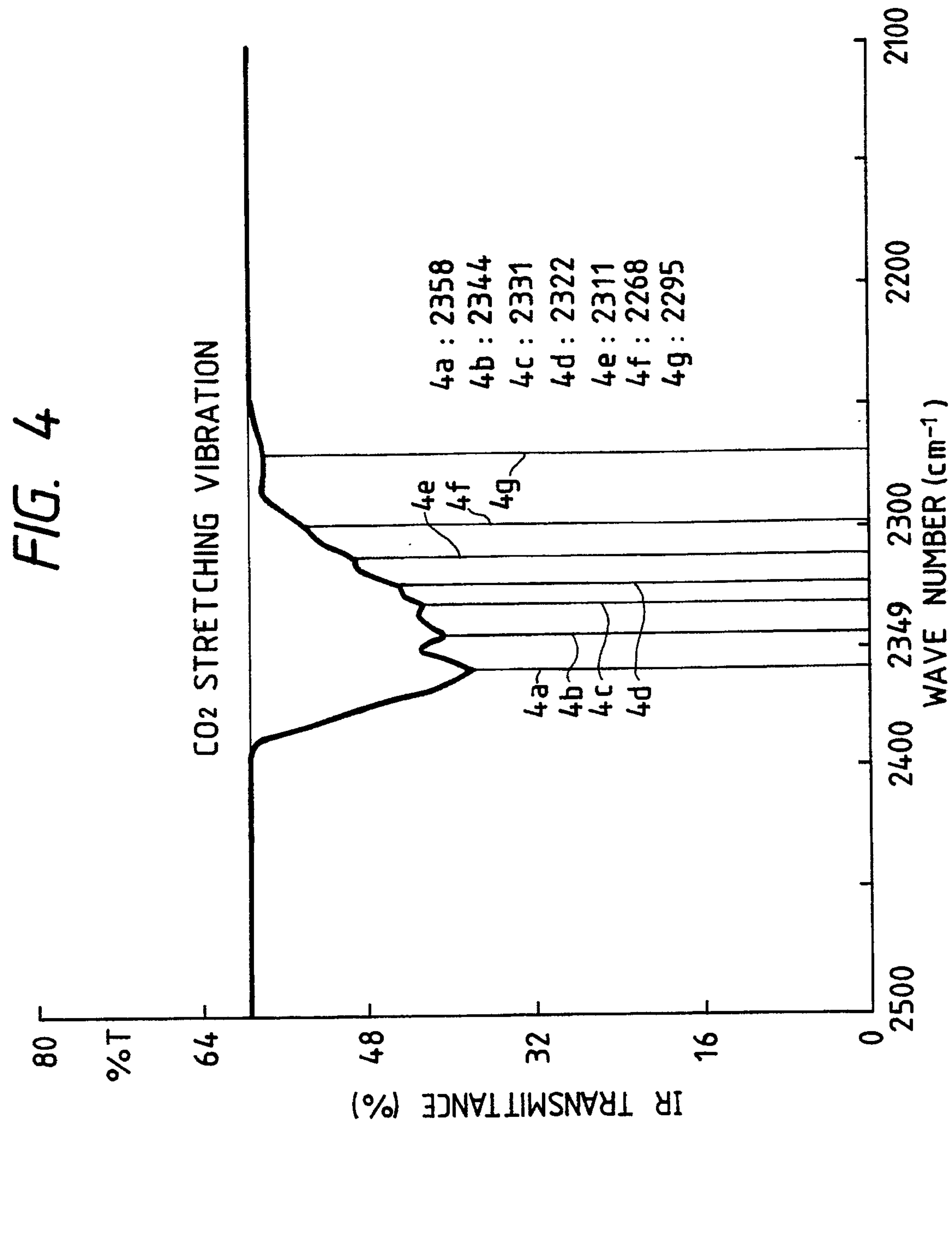

$CO_2$ STRETCHING VIBRATION
IR TRANSMITTANCE (%)
%T
80
64
48
32
16
0
2500
2400
2349
2300
2200
2100
WAVE NUMBER ($cm^{-1}$)
4a
4b
4c
4d
4e
4f
4g
4a : 2358
4b : 2344
4c : 2331
4d : 2322
4e : 2311
4f : 2268
4g : 2295

FIG. 6A

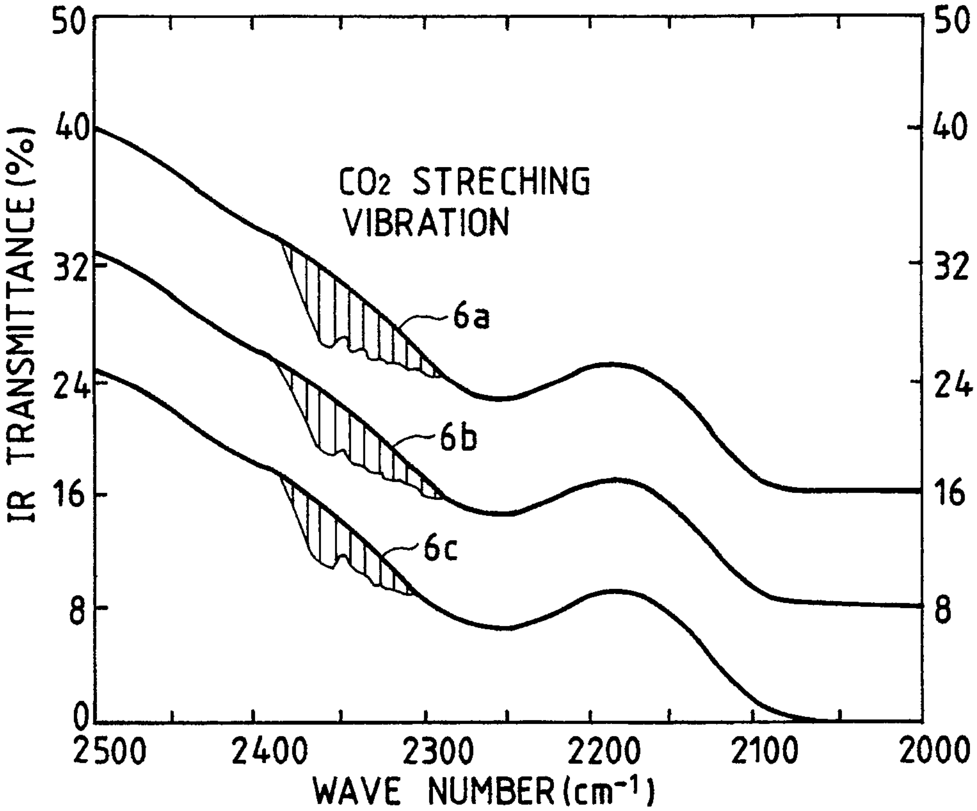
IR TRANSMITTANCE (%)
CO2 STRECHING VIBRATION
6a
6b
6c
50
40
32
24
16
8
0
2500
2400
2300
2200
2100
2000
WAVE NUMBER (cm-1)

FIG. 6B

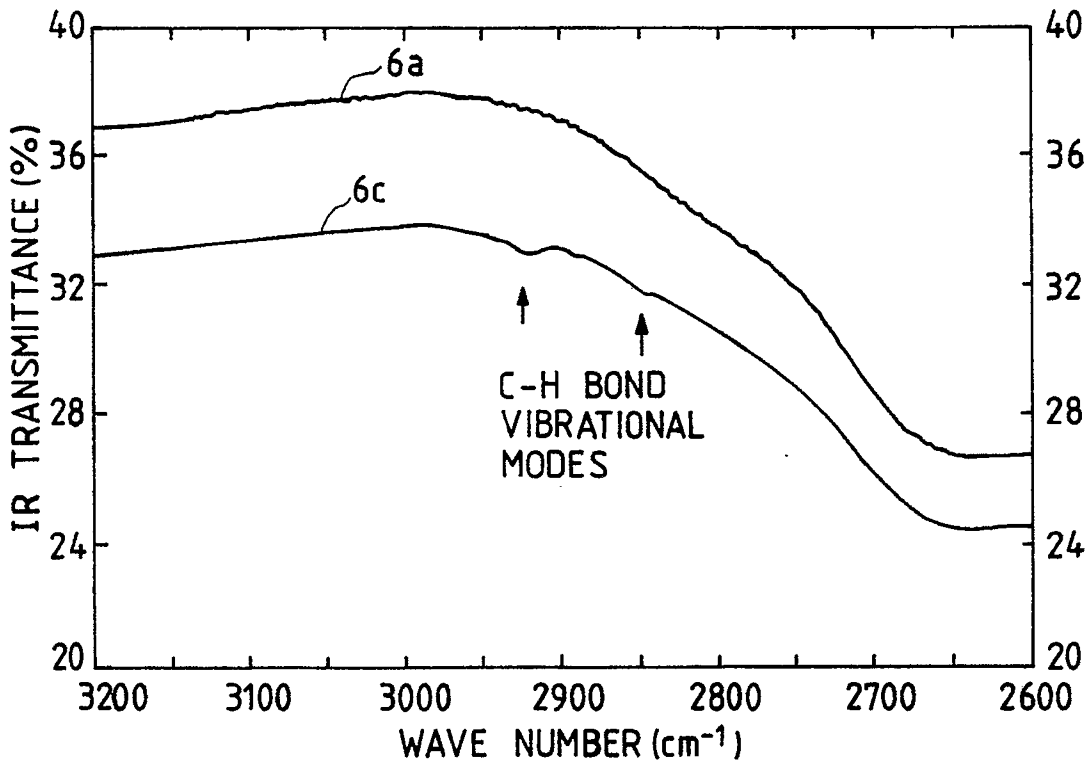
IR TRANSMITTANCE (%)
6a
6c
C-H BOND VIBRATIONAL MODES
40
36
32
28
24
20
3200
3100
3000
2900
2800
2700
2600
WAVE NUMBER (cm-1)

FIG. 7A

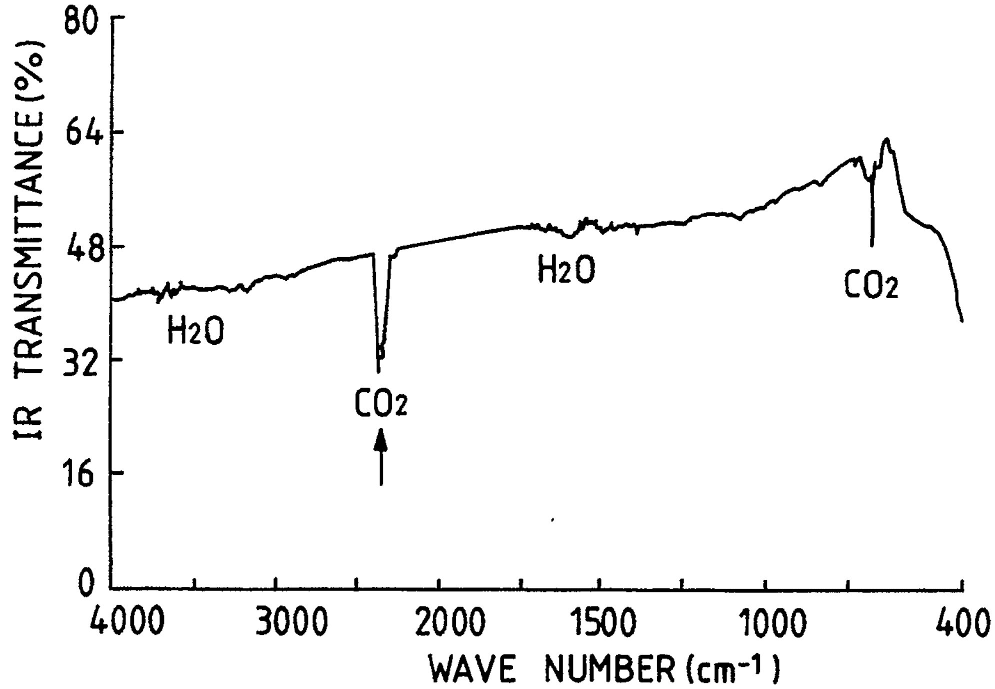
80
64
48
32
16
0
IR TRANSMITTANCE (%)
H2O
CO2
H2O
CO2
4000
3000
2000
1500
1000
400
WAVE NUMBER (cm-1)

FIG. 7B

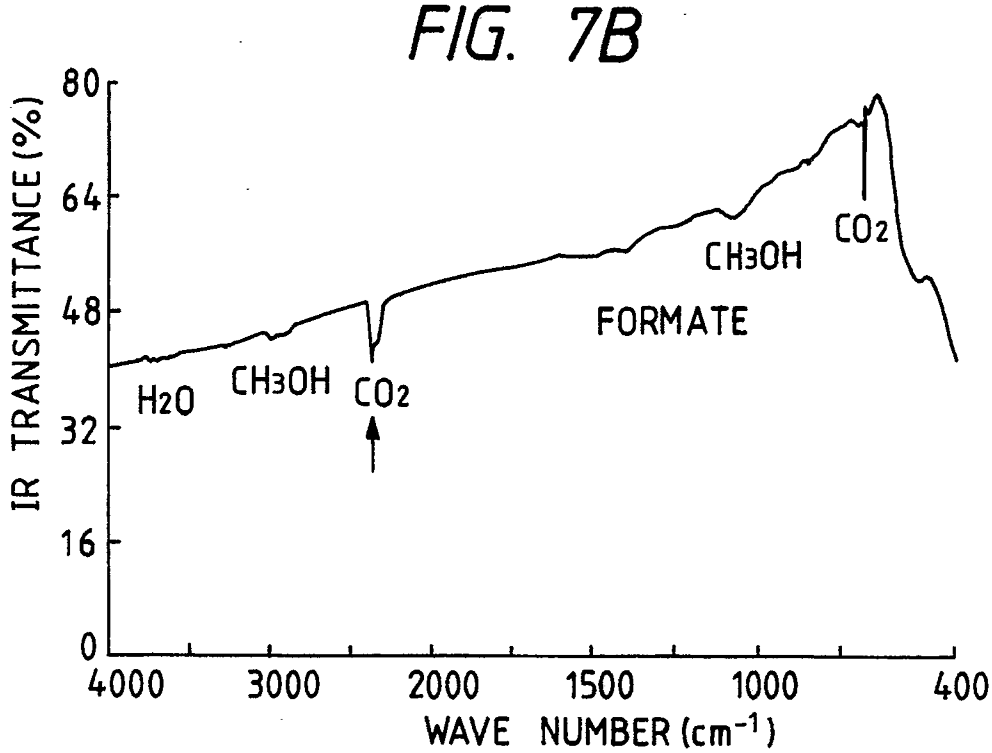
80
64
48
32
16
0
IR TRANSMITTANCE (%)
H2O
CH3OH
CO2
FORMATE
CH3OH
CO2
4000
3000
2000
1500
1000
400
WAVE NUMBER (cm-1)

FIG. 8

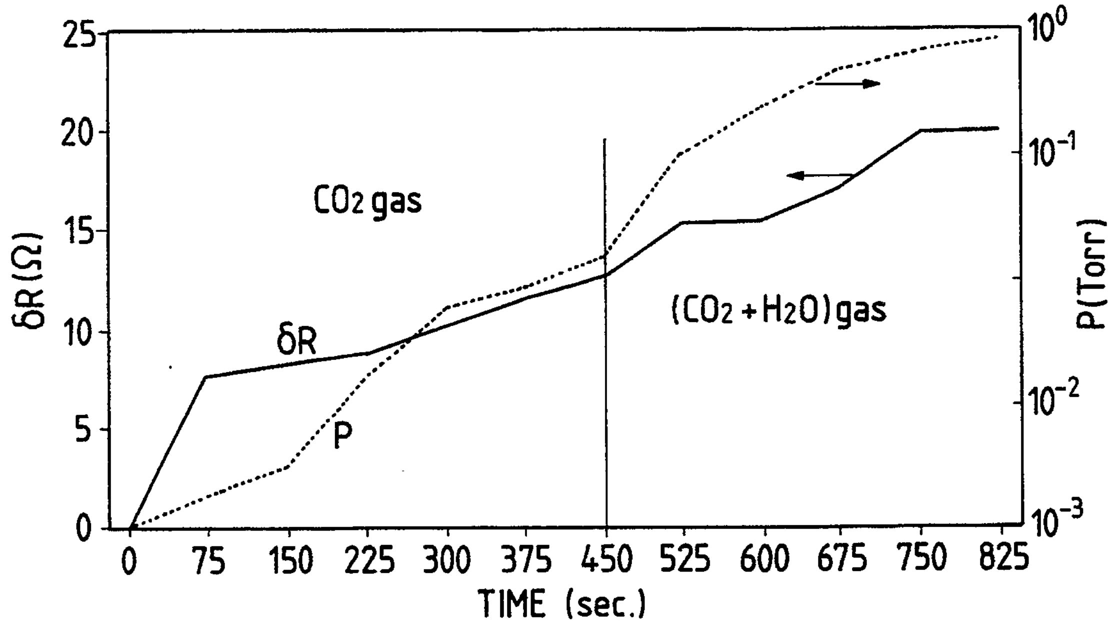


FIG. 9A

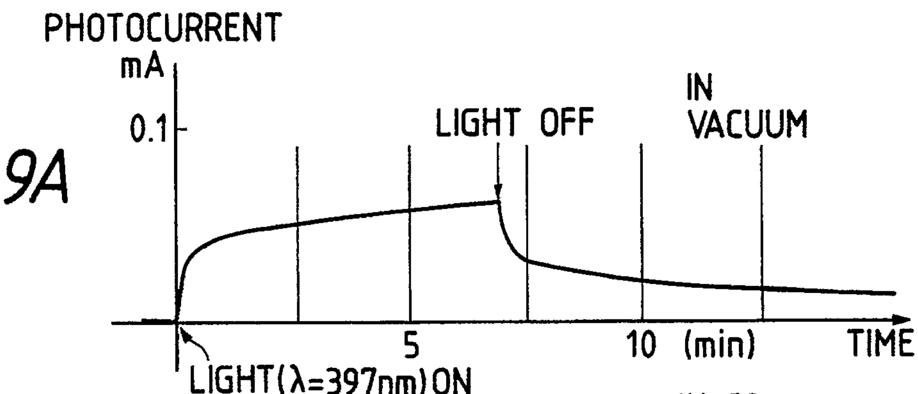


FIG. 9B

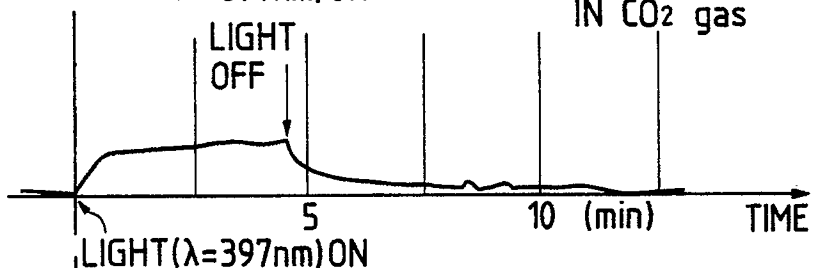


FIG. 9C

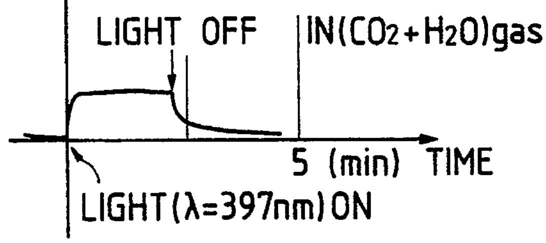

FIG. 10A

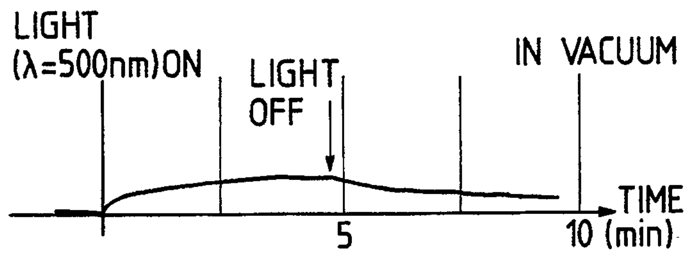


FIG. 10B

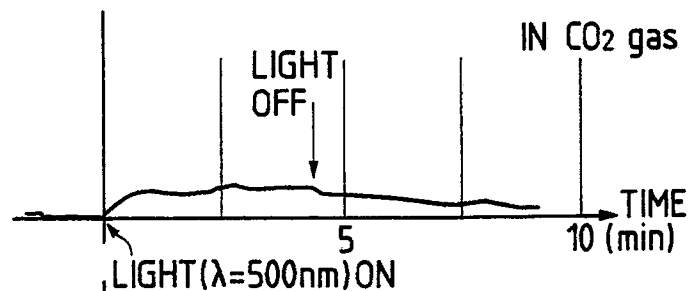


FIG. 10C

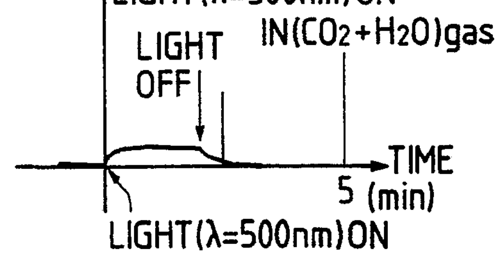


FIG. 11

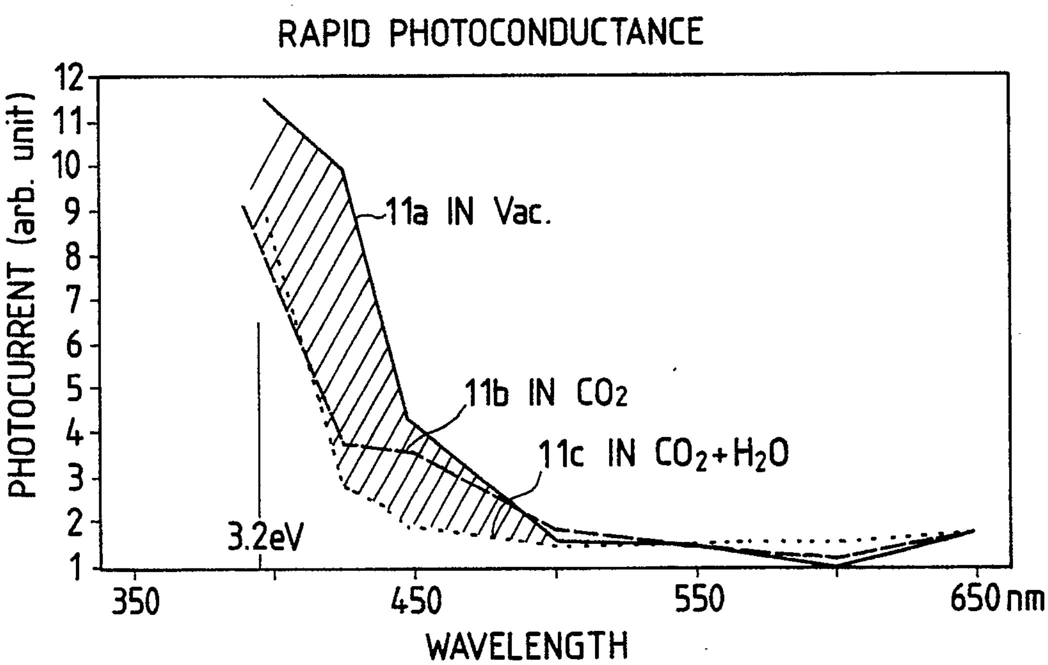

# FIG. 12

TABLE 1 : AUGER ANALYSIS OF CONSTITUENTS (%) ON ZnO SURFACE IN PERCENTS

| ELEMENT / SAMPLE | | Zn | O | C | S | P | Si |
|---|---|---|---|---|---|---|---|
| | AUGER SIGNAL ANALYZED (eV) | 990 | 510 | 273 | 152 | 122 | 90 |
| ZnO $(10\bar{1}0)$ | BEFORE ACTIVATION | 47.4 | 47.4 | 3.1 | 0.7 | 1.4 | 0.0 |
| ZnO POWDER (PRESSED) | BEFORE ACTIVATION | 38.0 | 46.7 | 4.7 | 0.0 | 2.5 | 8.1 |
| | AFTER ACTIVATION | 37.6 | 50.4 | 0.0 | 0.9% | 3.2% | 7.8% |
| | AFTER PHOTOREACTION WITH Xe LAMP | 33.3 | 45.3 | 5.5 | 1.6 | 3.9 | 10.4 |

FIG. 13

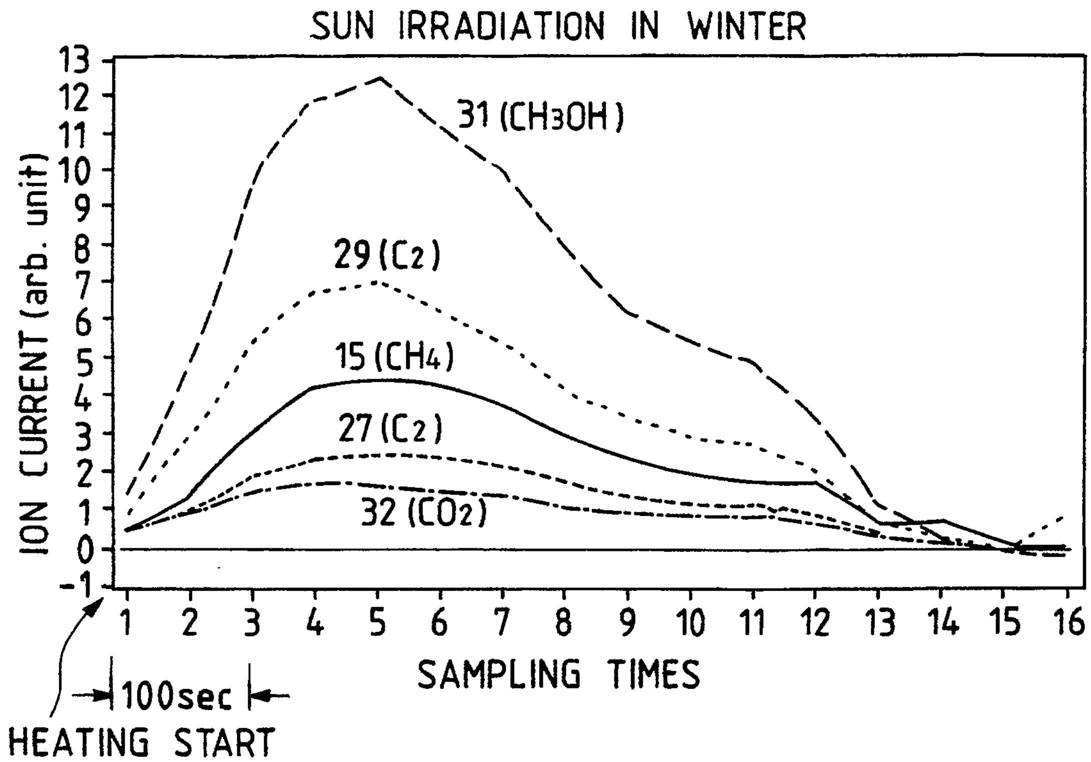
SUN IRRADIATION IN WINTER
ION CURRENT (arb. unit)
13
12
11
10
9
8
7
6
5
4
3
2
1
0
-1
31 (CH3OH)
29 (C2)
15 (CH4)
27 (C2)
32 (CO2)
1 2 3 4 5 6 7 8 9 10 11 12 13 14 15 16
SAMPLING TIMES
100sec
HEATING START

FIG. 14

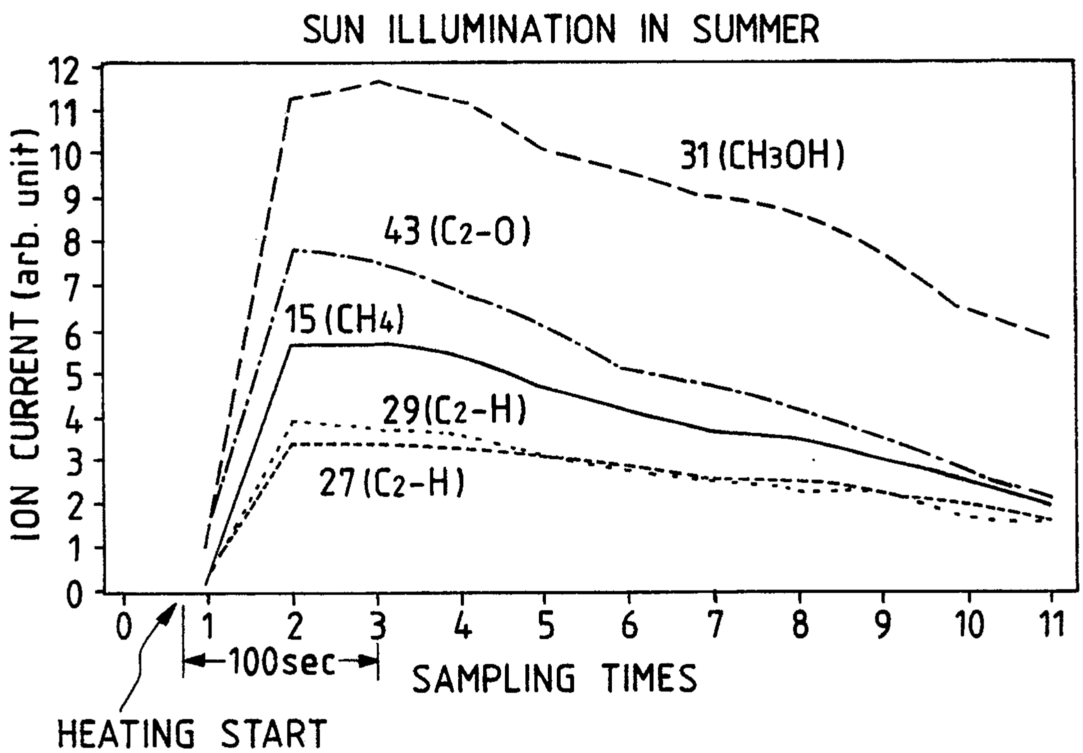
SUN ILLUMINATION IN SUMMER
ION CURRENT (arb. unit)
12
11
10
9
8
7
6
5
4
3
2
1
0
31 (CH3OH)
43 (C2-O)
15 (CH4)
29(C2-H)
27 (C2-H)
0 1 2 3 4 5 6 7 8 9 10 11
100sec
SAMPLING TIMES
HEATING START

FIG. 15

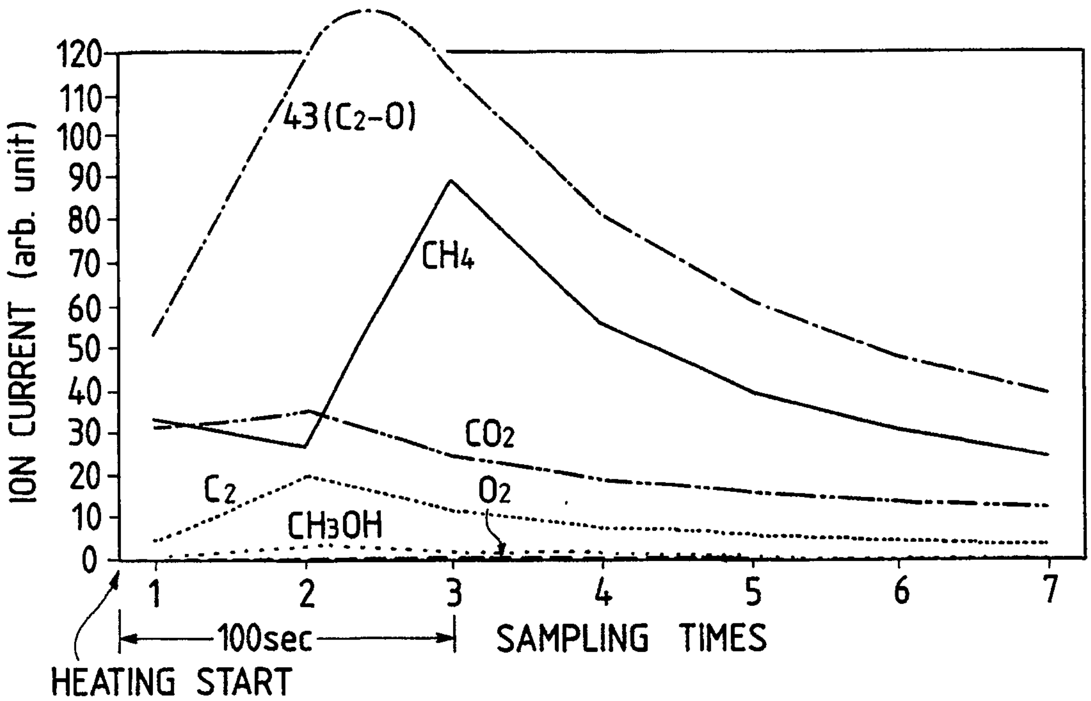
120
110
100
90
80
70
60
50
40
30
20
10
0
ION CURRENT (arb. unit)
43(C2-O)
CH4
CO2
C2
O2
CH3OH
1
2
3
4
5
6
7
100sec
SAMPLING TIMES
HEATING START

FIG. 16

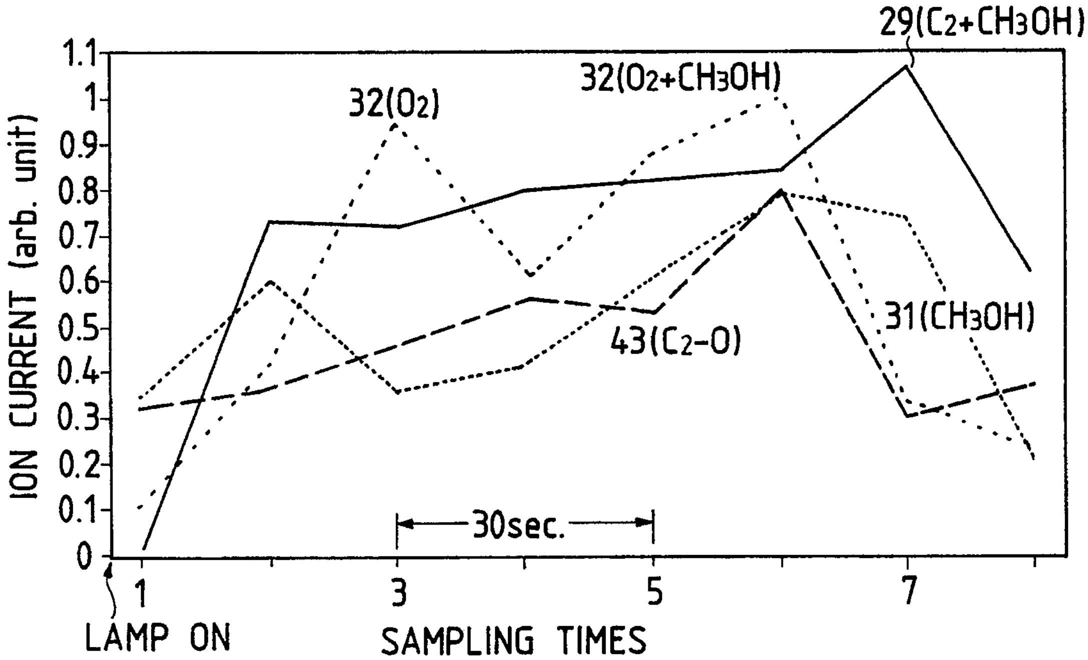
29(C2+CH3OH)
32(O2+CH3OH)
32(O2)
1.1
1
0.9
0.8
0.7
0.6
0.5
0.4
0.3
0.2
0.1
0
ION CURRENT (arb. unit)
31(CH3OH)
43(C2-O)
30sec.
1
3
5
7
LAMP ON
SAMPLING TIMES

FIG. 17

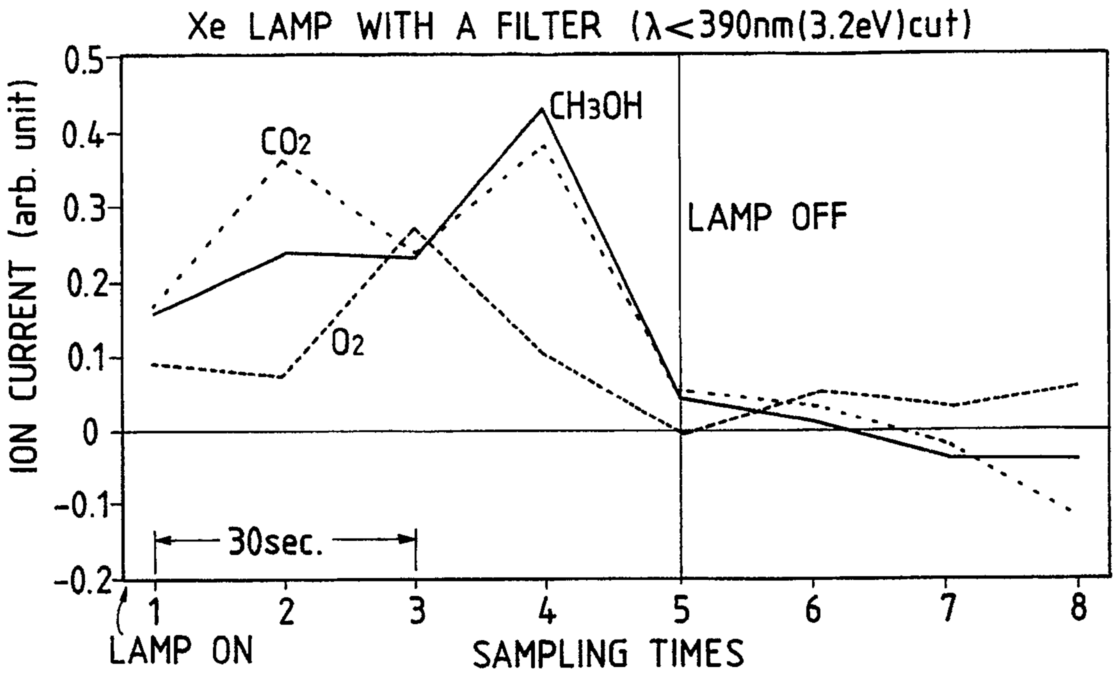
Xe LAMP WITH A FILTER (λ<390nm(3.2eV)cut)
ION CURRENT (arb. unit)
0.5
0.4
0.3
0.2
0.1
0
-0.1
-0.2
CO2
CH3OH
O2
LAMP OFF
30sec.
1
2
3
4
5
6
7
8
LAMP ON
SAMPLING TIMES

FIG. 18

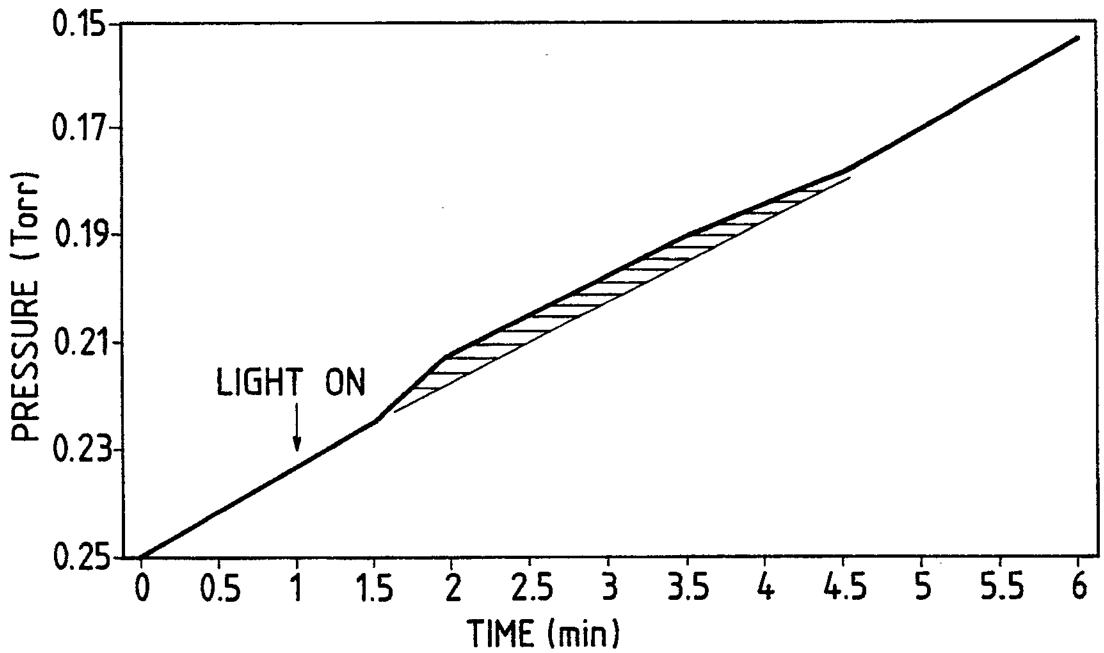
PRESSURE (Torr)
0.15
0.17
0.19
0.21
0.23
0.25
LIGHT ON
0
0.5
1
1.5
2
2.5
3
3.5
4
4.5
5
5.5
6
TIME (min)

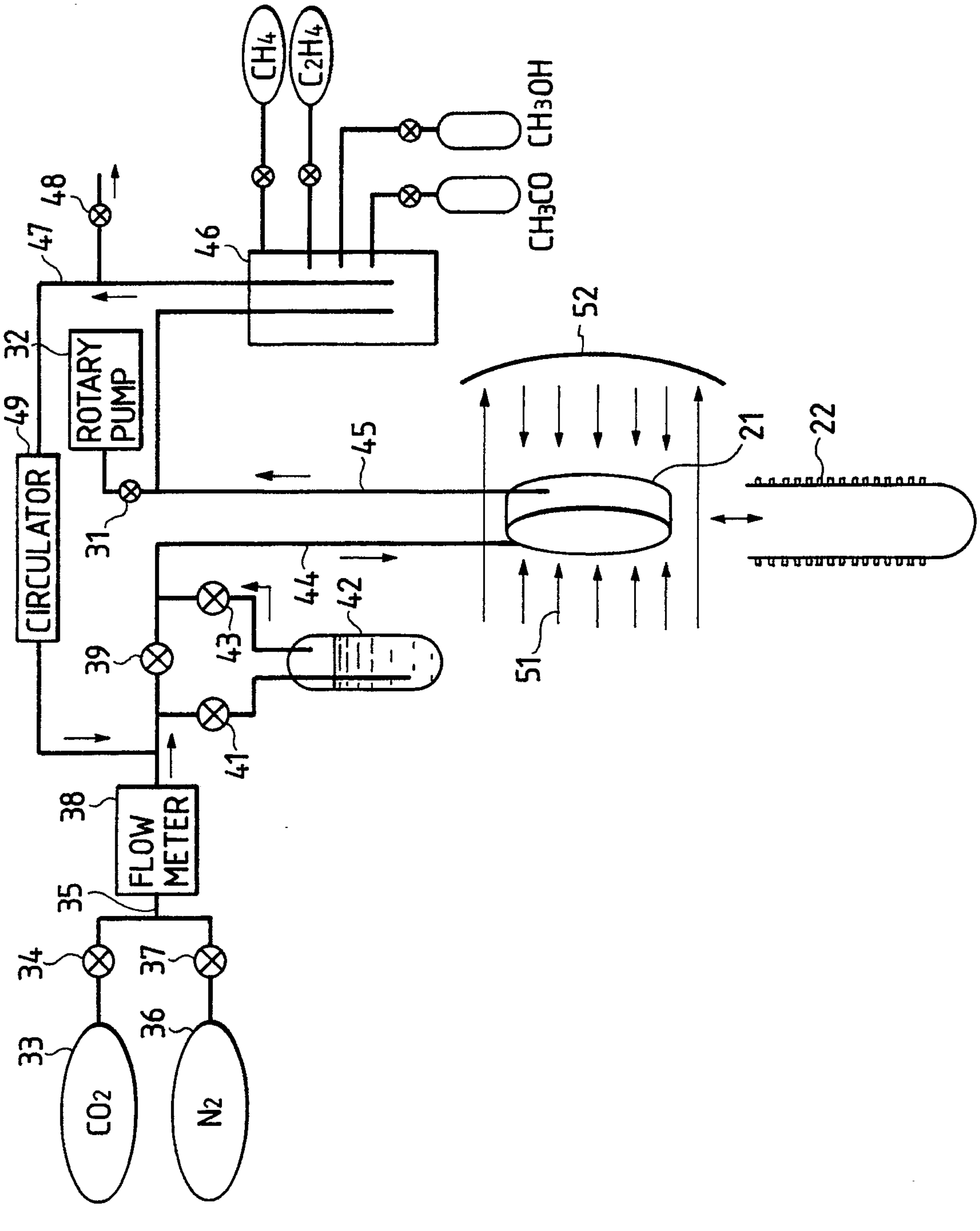
CO2
N2
33
36
34
37
35
FLOW METER
38
CIRCULATOR
49
ROTARY PUMP
32
31
39
41
42
43
44
45
46
47
48
CH4
C2H4
CH3CO
CH3OH
21
22
51
52

FIG. 19

European Patent Office

# EUROPEAN SEARCH REPORT

Application Number

EP 91108837.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | CHEMICAL ABSTRACTS, vol. 94, no. 16, April 20, 1981, Columbus, Ohio, USA B. AURIAN-BLAJENI "Photo-reduction of carbon dioxide and water into formaldehyde and methanol on semiconductor materials" page 182, abstract-no. 124 490v & Sol. Energy 1980, 25(2), 165-70 | 1,2,3, 5,9 | C 07 C 29/15<br>C 07 C 31/04<br>C 07 C 27/00<br>B 01 J 19/08 |
| Y | US - A - 4 219 392 (HALMANN) * Example 5; claims 2,3 * | 1,2,3, 5,9 | |
| D,A | NATURE, vol. 277, February 22, 1979, London TOORU INOUE et al. "Photo-electrocatalytic reduction of carbon dioxide in aqueous suspensions of semiconductor powders" pages 637,638 * Totality * | 1,4,7, 9,10 | TECHNICAL FIELDS SEARCHED (Int. Cl.5)<br>C 07 C 29/00<br>C 07 C 27/00<br>C 07 C 31/00<br>C 07 C 53/00<br>C 07 C 1/00<br>B 01 J |
| A | CHEMICAL ABSTRACTS, vol. 97, no. 26, December 27, 1982, Columbus, Ohio, USA M. ULMAN et al. "Photoassisted carbon dioxide reduction to organic compounds using rare earth-doped barium titanate and lithium niobate as photo-active agents" page 768, abstract-no. 227 344s & Isr.J.Chem. 1982, 22(2), 177-9 | 1 | |

The present search report has been drawn up for all claims

| Place of search | Date of completion of the search | Examiner |
|---|---|---|
| VIENNA | 14-08-1991 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-

EP 91108837.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP - A2/A3 - 0 070 712 (SIBIT S.P.A.) * Example 5 * | 1,6 | |
| | | | TECHNICAL FIELDS SEARCHED (Int. Cl.5) |

The present search report has been drawn up for all claims

| Place of search | Date of completion of the search | Examiner |
|---|---|---|
| VIENNA | 14-08-1991 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)